(19) Europäisches Patentamt
European Patent Office
Office européen des brevets

(11) Publication number: **0 077 028**
**B1**

(12) # EUROPEAN PATENT SPECIFICATION

(45) Date of publication of patent specification: **14.01.87**

(51) Int. Cl.[4]: **C 07 K 7/02,** A 61 K 37/02, A 61 K 37/64

(21) Application number: **82109271.5**

(22) Date of filing: **07.10.82**

(54) **Renin inhibitory peptides.**

(30) Priority: **08.10.81 US 309855**
**19.10.81 US 312558**

(43) Date of publication of application:
**20.04.83 Bulletin 83/16**

(45) Publication of the grant of the patent:
**14.01.87 Bulletin 87/03**

(84) Designated Contracting States:
**AT BE CH DE FR GB IT LI LU NL SE**

(56) References cited:
**FR-A-2 262 532**
**FR-A-2 399 409**

(73) Proprietor: **MERCK & CO. INC.**
**126, East Lincoln Avenue P.O. Box 2000**
**Rahway New Jersey 07065 (US)**

(72) Inventor: **Veber, Daniel F.**
**290 Batleson Road**
**Ambler Pennsylvania 19002 (US)**
Inventor: **Boger, Joshua S.**
**719 New Gulph Road**
**Bryn Mawr Pennsylvania 19010 (US)**

(74) Representative: **Abitz, Walter, Dr.-Ing. et al**
**Abitz, Morf, Gritschneder, Freiherr von Wittgenstein Postfach 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 077 028 B1

**Description**

BACKGROUND OF THE INVENTION

1. Field of the Invention

The present invention is concerned with novel peptides with inhibit renin.

The present invention is also concerned with pharmaceutical compositions containing the novel peptides of the present invention as active ingredients, with methods of treating renin-associated hypertension and hyperaldosteonism, with diagnostic methods which utilize the novel peptides of the present invention, and with methods of preparing the novel peptides of the present invention.

Renin is a proteolytic enzyme of molecular weight about 40,000, produced and secreted by the kidney. It is secreted by the juxtaglomerular cells and acts on the plasma substrate, angiotensinogen, to split off the decapeptide angiotensin I, which is converted to the potent pressor agent angiotensin II. Thus, the renin-angiotensin system plays an important role in normal cardiovascular homeostasis and in some forms of hypertension.

In the past, attempts to modulate or manipulate the renin-angiotensin system have met with success in the use of inhibitors of angiotensin I converting enzyme. In view of this success, it seems reasonable to conclude that a specific inhibitor of the limiting enzymatic step that ultimately regulates angiotension II production, the action of renin on its substrate, would be at least equally successful. Thus, an effective inhibitor of renin has been long sought as both a therapeutic agent and as an investigative tool.

2. Brief Description of the Prior Art

There has been substantial interest in the synthesis of useful renin inhibitors for many decades; and the following table lists the major classes of renin inhibitors that have been studied, as well as their inhibition constants ($K_I$):

| Class | $K_I$ (M) |
|---|---|
| Renin antibody | probably $10^{-6}$ |
| Pepstatin | $10^{-6} - 10^{-7}$ |
| Phospholipids | $10^{-3}$ |
| Substrate analogs | |
| Tetrapeptides | $10^{-3}$ |
| Octa- to tridecapeptides | $10^{-5} - 10^{-6}$ |

Umezawa et al., in *J. Antibiot. (Tokyo)* 23: 259—262, 1970, reported the isolation of a peptide from actinomyces that was an inhibitor of aspartyl proteases such as pepsin, cathepsin D, and renin. This peptide, known as pepstatin, was found by Gross et al., *Science* 175:656, 1971, to reduce blood pressure in vivo after the injection of hog renin into nephrectomized rats. However, pepstatin has not found wide application as an experimental agent because of its limited solubility and its inhibition of a variety of other acid proteases in addition to renin. The structure of pepstatin is shown below:

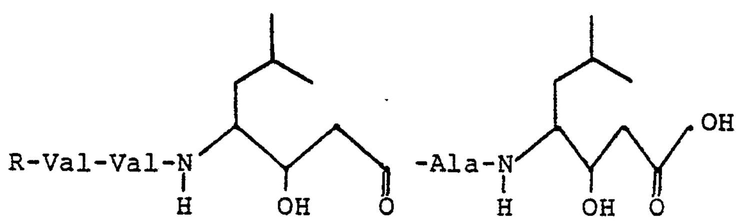

To date, many efforts have been made to prepare a specific renin inhibitor based on substrate analogy. Since the human renin substrate has only recently been elucidated (Tewksbury et al., *Circulation* 59, 60, Supp. II: 132, Oct. 1979), heretofore substrate analogy has been based on the known pig renin substrate. While the human and pig renin substrates are not the same, the substrate analogy based on pig renin has always been considered acceptable in the art as predictive of human renin inhibitory activity because of the closely related activity of the two renins. Thus, while pig renin does not cleave the human renin substrate, human renin, on the other hand, does cleave the pig renin substrate. See Pulsen et al., *Biochim. Biophys. Acta* 452:533—537, 1976; and Skeggs, Jr. et al., *J. Exp. Med.* 106:439—453, 1957.

It has been found, for example, using pig renin substrate analogy, that the octapeptide sequence extending from histidine-6 through tyrosine-13 has kinetic parameters essentially the same as those of the full tetradecapeptide renin substrate. The amino acid sequence of the octapeptide in pig renin substrate is as follows:

6 7 8 9 10 11 12 13
-His-Pro-Phe-His-Leu-Leu-Val-Tyr-

Renin cleaves this substrate between $Leu^{10}$ and $Leu^{11}$.

The renin inhibitory peptides of the present invention are also based on human renin substrate analogy. The human renin octapeptide sequence, related to the pig renin substrate octapeptide, is as follows:

6 7 8 9 10 11 12 13
-His-Pro-Phe-His-Leu-Val-Ile-His-

Similar to the pig renin substrate, human renin cleaves thus substrate between $Leu^{10}$ and $Val^{11}$.

Kokubu et al., *Biochem. Pharmacol.* 22: 3217—3223, 1973, synthesized a number of analogs of the tetrapeptide found between residues 10 to 13 of the pig renin substrate, but while inhibition could be shown, inhibitory constants were only of the order of $10^{-3}$M.

Analogs of a larger segment of renin substrate were also synthesized: Burton et al., *Biochemistry* 14: 3892—3898, 1975, and Poulsen et al., *Biochemistry* 12: 3877—3882, 1973. Two of the major obstacles which had to be overcome to obtain an effective renin inhibitor useful in vivo were lack of solubility and weak binding (large inhibitory constant). Modifications to increase solubility soon established that the inhibitory properties of the peptides are markedly dependent on the hydrophobicity of various amino acid residues, and that increasing solubility by replacing lipophilic amino acids with hydrophilic isosteric residues becomes counter-productive. Other approaches to increasing solubility have had limited success. Various modifications designed to increase binding to renin have also been made, but here too, with only limited success. For a more detailed description of past efforts to prepare an effective inhibitor of renin, see Haber and Burton, *Fed. Proc. Fed. Am. Soc. Exp. Biol.* 38: 2768—2773, 1979.

For other articles describing previous efforts to devise renin inhibitors, see Marshall, *Federation Proc.* 35: 2494—2501, 1976; Burton et al., *Proc. Natl. Acad. Sci. USA* 77: 5476—5479, Sept. 1980; Suketa et al., *Biochemistry* 14: 3188, 1975; Swales, *Pharmac. Ther.* 7: 173—201, 1979; Kokubu et al., *Nature* 217; 456—457, Feb. 3, 1968; Matsushita et al., *J. Antibiotics* 28: 1016—1018, Dec. 1975; Lazar et al., *Biochem. Pharma.* 23: 2776—2778, 1974; Miller et al., *Biochem. Pharma.* 21: 2941—2944, 1972; Haber, *Clinical Science* 59:7s—19s, 1980; and Rich et al., *J. Org. Chem.* 43: 3624, 1978, and *J. Med. Chem.* 23: 27, 1980.

FR—A—2 399 409 described peptides which differ from the present peptides in that they bear at the carboxyl end of the molecule a residue of the formula $—Sta—(X)_a—(Y)_b—(Z)_c$ wherein X, Y and Z represent an amino acid chosen from arginine, glutamic acid, lysine, histidine and valine and the sum of a + b + c may be 1, 2 or 3. There is no pointer to the present specific peptides nor to their improved renin inhibiting effect.

DETAILED DESCRIPTION OF THE INVENTION AND PREFERRED EMBODIMENTS

In accordance with the present invention there are provided renin inhibitory peptides of the formula:

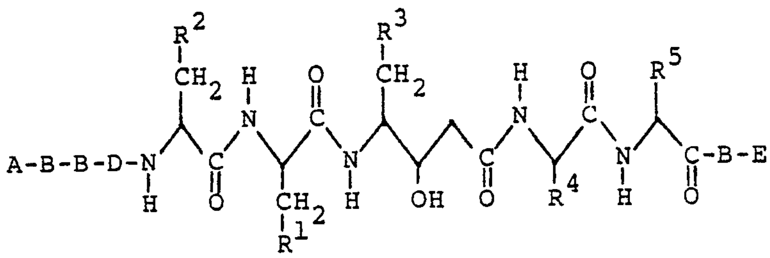

(I.)

wherein:

A is hydrogen;

$$R^3—O—CH_2—\overset{O}{\overset{\|}{C}}—;\quad R^3—CH_2—O—\overset{O}{\overset{\|}{C}}—;\quad R^3—O—\overset{O}{\overset{\|}{C}}—;\quad \text{or } R^3—(CH_2)_n—\overset{O}{\overset{\|}{C}}—,$$

where n is 0 to 5 and $R^3$ has the same meaning as set out further below, and may additionally be hydrogen;

B is absent; glycyl; sarcosyl; or

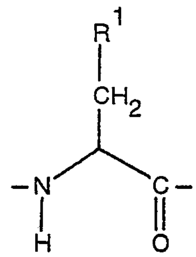 ;

D is absent; or

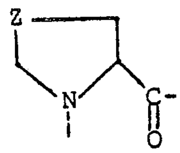

where Z is $(CH_2)_n$ and n is 1 or 2; or —S—;

$R^1$ is hydrogen; $C_{1-4}$ alkyl; hydroxy $C_{1-4}$ alkyl; phenyl; phenyl mono-substituted with a member selected fron the group consisting of methyl, trifluoromethyl, hydroxy, methoxy, fluoro, chloro, bromo, and iodo; indolyl; 4-imidazolyl; amine $C_{2-4}$ alkyl; guanidyl $C_{2-3}$ alkyl; or methylthiomethyl;

$R^2$ is hydrogen; $C_{1-4}$ alkyl; phenyl; phenyl mono-substituted with a member selected from the group consisting of methyl, trifluoromethyl, hydroxy, methoxy, fluoro, chloro, bromo, and iodo; or indolyl;

$R^3$ is $C_{3-6}$ alkyl; $C_{3-7}$ cycloalkyl; phenyl; or phenyl mono-substituted with a member selected from the group consisting of methyl, trifluoromethyl, hydroxy, methoxy, fluoro, chloro, bromo, and iodo;

$R^4$ is hydogen; or

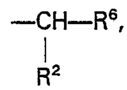

where $R^6$ is hydrogen; $C_{1-4}$ alkyl; hydroxy; or $C_{3-7}$ cycloalkyl;

$R^5$ is hydrogen;

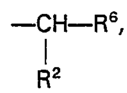

where $R^6$ is hydrogen, $C_{1-4}$ alkyl, hydroxy, or $C_{3-7}$ cycloalkyl; or $—CH_2—R^7$, where $R^7$ is 4-imidazolyl, amino $C_{2-4}$ alkyl, 2-, 3- or 4-pyridyl or guanidyl $C_{2-3}$ alkyl; and

E is $OR^8$; $NHR^8$, or $N(R^8)_2$, where each $R^8$ may be the same or different and is hydrogen or $C_{1-4}$ alkyl; wherein all of the asymmetric carbon atoms have an *S* configuration, except those of substituents B and D, which may also have the *R* configuration; and a pharmaceutically acceptable salt thereof.

In the above definitions, the term "alkyl" is intended to include both branched and straight chain hydrocarbon groups having the indicated number of carbon atoms.

The novel renin inhibitory peptides of the present invention may also be described in terms of common amino acid components and closely related analogs thereof, in accordance with the following formula:

$$A—B—B—D—F—G—Sta—H—I—B—E \quad (II.)$$

The A, B, D, and E components correspond to the same portions of Formula I.

In Formula II, Sta represents the unusual amino acid statine and its closely related analogs, and its presence constitutes a unique feature of the renin inhibitory peptides of the present invention. Statine may be named as 4(S)-amino-3(S)-hydroxy-6-methylheptanoic acid, and may be represented by the following formula:

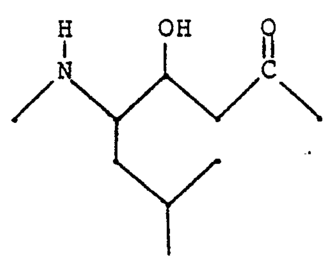

(III)

As shown in Formula III above, the delta-substituent in naturally-occurring statine is isoproyl, or a leucine sidechain, essentially. As shown in Formula I by the $R^3$ substituents, the isopropyl group may be replaced by higher alkyl groups up to six carbon atoms, cycloalkyl groups containing from three to seven carbon atoms, phenyl, and phenyl mono-substituted with a member selected from the group consisting of methyl, trifluoromethyl, hydroxy, methoxy, fluoro, chloro, bromo, and iodo. The phenyl substituent is especially preferred. These modifications of the naturally-occurring statine structure are in accordance with the hydrophobicity considered necessary to maintain the inhibitory activity of the total peptide.

The remaining common amino acid components of Formula II are as follows:

A has the same meaning as above in Formula I;

B is absent, Gly, Ala, Val, Leu, Ile, Phe, Tyr, Trp, His, Lys, Orn, Arg, or Met;

D is absent or Pro;

F is Ala, Leu, Phe, Tyr, or Trp;

G is Ala, Leu, Phe, Tyr, Trp, His, Lys, Orn, Arg, or Met;

H is the same as F and may additionally be Ser, Gly, Val, Ile, or Thr;

I is the same as H and may additionally be His, Arg, Lys, or Orn; and

E has the same meaning as above in Formula I.

It will be understood that closely related analogs of the above common amino acids, for example, aliphatic amino acids in addition to Ala, Val, Leu, and Ile, such as α-aminobutyric acid (Abu), and substituted phenyl derivatives of Phe, are included in the broad description of the novel inhibitory peptides of the present invention represented by Formula I and its definitions. Thus, the peptides of Formula II and its definitions, including the derivatives of naturally-occurring statine represented by the definitions of the $R^3$ substituent in Formula I, represent preferred peptides of the present invention.

Especially preferred inhibitory peptides of the present invention are the following:

*iso*-Butyryl-His-Pro-Phe-His-Sta-Val-His-Gly-$NH_2$
*iso*-Butyryl-His-Pro-Phe-His-Sta-Ile-His-$NH_2$
*tert*-Butyloxycarbonyl-Phe-His-Sta-Ile-His-$NH_2$
Benzyloxycarbonyl-Phe-His-Sta-Ile-His-$NH_2$
*iso*-Valeryl-His-Pro-Phe-His-Sta-Ile-His-$NH_2$
*iso*-Valeryl-His-Pro-Phe-His-Sta-Leu-His-$NH_2$
His-Pro-Phe-His-Sta-Leu-Phe-$NH_2$
*iso*-Valeryl-His-Pro-Phe-His-Sta-Leu-Phe-$NH_2$
Acetyl-Pro-Phe-His-Sta-Leu-Phe-$NH_2$
Acetyl-Phe-His-Sta-Leu-Phe-$NH_2$
*tert*-Butyloxycarbonyl-Phe-His-Sta-Leu-Phe-$NH_2$
*tert*-Butyloxycarbonyl-His-Pro-Phe-Phe-Sta-Leu-Phe-$NH_2$
*iso*-Butyryl-His-Pro-Phe-His-Sta-Ala-Phe-$NH_2$
*iso*-Butyryl-His-Pro-Phe-His-Sta{Cyclo-hexyl-Ala}-Phe-$NH_2$
*tert*-Butyloxycarbonyl-His-Pro-Phe-His-Sta-Leu-Leu-$OCH_3$
*tert*-Butyloxycarbonyl-His-Pro-Phe-His-Sta-Leu-Tyr-$NH_2$
*iso*-Butyryl-His-Pro-Phe-His-Sta-Leu-Phe-Lys-$NH_2$
*tert*-Butyloxycarbonyl-His-Pro-Phe-*p*-I-Phe-Sta-Leu-Phe-$NH_2$
*iso*-Valeryl-His-Pro-Phe-His-Sta-Leu-Val-Phe-$NH_2$

The inhibitory peptides of the present invention may be better appreciated in terms of substrate analogy from the following illustration of Formula I alongside the octapeptide sequence of a portion of the pig renin substrate, which renin cleaves between $Leu^{10}$ and $Leu^{11}$:

His-Pro Phe His Leu Leu Val Tyr
(5) 6 7 . 8 9 10(11) 12 13 (14)

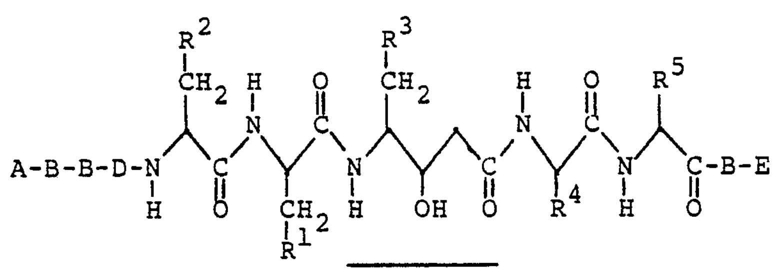

Statine

(IV.)

As can be seen, a unique aspect and essential feature of the present invention is the substitution of the single statine amino acid component for the double amino acid sequence: $Leu^{10}$-$Leu^{11}$ in the endogenous

pig renin substrate. It is believed that substitution of statine for both leucine amino acids rather than just one leucine results in an improved substrate analogy due to the greater linear extent of statine as compared to a single leucine component. Thus, statine more closely approximates Leu-Leu in linear extent, and thereby provides a better "fit" to the renin enzyme.

The inhibitory peptides of the present invention may also be better appreciated in terms of substrate analogy from the following illustration of Formula I alongside the octapeptide sequence of a portion of the human renin substrate, which renin cleaves between $Leu^{10}$ and $Val^{11}$:

His-Pro-Phe His Leu Val Ile His
(5) 6 7 8 9 10(11) 12 13 (14)

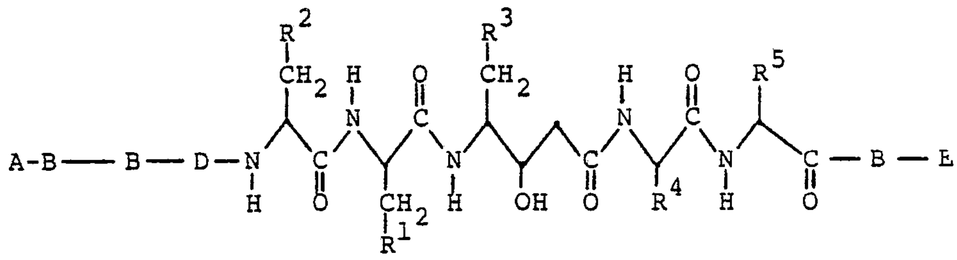

Statine

(V.)

As can be seen, a unique aspect and essential feature of the present invention is the substitution of the single statine amino acid component for the double amino acid sequence: $Leu^{10}$-$Val^{11}$ in the endogenous human renin substrate. It is believed that substitution of statine for both the leucine and valine amino acids rather than just the leucine results in an improved substrate analogy due to the greater linear extent of statine as compared to a single leucine component. Thus, statine more closely approximates Leu-Val in linear extent, and thereby provides a better "fit" to the human renin enzyme.

In the endogenous substrate it is also preferred to substitute Leu for $Val^{12}$ and Phe for $Tyr^{13}$ in order to enhance the inhibitory activity of the resulting peptide. Thus, a most preferred inhibitory peptide of the present invention is His-Pro-Phe-His-Sta-Leu-Phe-$NH_2$.

Other specific preferred peptides of the present invention are the following, set out in order by variations in the structure at different positions in the overall peptide sequence, represented by the numbered positions in Formulas IV and V:

| Variations in position (5): | | | | | 10 | | | |
|---|---|---|---|---|---|---|---|---|
| (5) | 6 | 7 | 8 | 9 | (11) | 12 | 13 | (14) |
| Hydrogen- | His— | Pro— | Phe— | His— | Sta— | Leu— | Phe— | $NH_2$ |
| *tert*-Butyloxycarbonyl- | " | " | " | " | " | " | " | " |
| *iso*-Valeryl- | " | " | " | " | " | " | " | " |
| *iso*-Butyryl- | " | " | " | " | " | " | " | " |
| *tert*-Butyloxycarbonyl- | " | " | " | " | " | " | " | " |
| Pro- | " | " | " | " | " | " | " | " |
| Hydrogen- | Sar— | Pro— | Phe— | His— | Sta— | Lue— | Phe— | $NH_2$ |
| Acetyl | X*– | " | " | " | " | " | " | " |
| *tert*-Butyloxycarbonyl | " | " | " | " | " | " | " | " |
| Phenoxyacetyl | " | " | " | " | " | " | " | " |
| Hydrogen | X—— | X– | " | " | " | " | " | " |

Variations in position (5):

| (5) | 6 | 7 | 8 | 9 | 10 (11) | 12 | 13 | (14) |
|---|---|---|---|---|---|---|---|---|
| *tert*-Butyloxycarbonyl | X— | —X— | —Phe— | —His— | —Sta— | —Leu— | —Phe— | —$NH_2$ |
| Cyclopentylcarbonyl | " | " | " | " | " | " | " | " |
| Acetyl | " | " | " | " | " | " | " | " |

Variations in position (6):

| (5) | 6 | 7 | 8 | 9 | 10 (11) | 12 | 13 | (14) |
|---|---|---|---|---|---|---|---|---|
| Hydrogen- | Sar— | —Pro— | —Phe— | —His— | —Sta— | —Leu— | —Phe— | —$NH_2$ |
| " | D–His– | " | " | " | " | " | " | " |

Variations in position (7):

| (5) | 6 | 7 | 8 | 9 | 10 (11) | 12 | 13 | (14) |
|---|---|---|---|---|---|---|---|---|
| | | –D– | | | | | | |
| *iso*-Valeryl | His— | —Pro— | —Phe— | —His— | —Sta— | —Leu— | —Phe— | —$NH_2$ |

Variations in position (8):

| (5) | 6 | 7 | 8 | 9 | 10 (11) | 12 | 13 | (14) |
|---|---|---|---|---|---|---|---|---|
| *iso*-Butyryl | His— | —Pro— | —Ala— | —Phe— | —Sta— | —Leu— | —Phe— | —$NH_2$ |
| | | | –*o*–I– | | | | | |
| tert-Butyloxycarbonyl- | " | " | Phe— | —His– | " | " | " | " |

Variations in position (9):

| (5) | 6 | 7 | 8 | 9 | 10 (11) | 12 | 13 | (14) |
|---|---|---|---|---|---|---|---|---|
| *iso*-Butyryl- | His— | —Pro— | —Phe— | —Ala— | —Sta— | —Leu— | —Phe— | —$NH_2$ |
| *tert*-Butyloxycarbonyl- | " | " | " | –Phe– | " | " | " | " |
| | | | | –*p*–I– | | | | |
| " | " | " | " | Phe– | " | " | " | " |

Variations in position (12):

| (5) | 6 | 7 | 8 | 9 | 10 (11) | 12 | 13 | (14) |
|---|---|---|---|---|---|---|---|---|
| *iso*-Butyryl- | His— | —Pro— | —Phe— | —His— | —Sta— | —Gly— | —Phe— | —$NH_2$ |
| " | " | " | " | " | " | –Ala– | " | " |
| " | " | " | " | " | " | –Val– | " | " |
| " | " | " | " | " | " | –Ile– | " | " |
| " | " | " | " | " | " | –Phe– | " | " |
| | | | | | | –Cyclohexyl– | | |
| " | " | " | " | " | " | Ala– | " | " |

Variations in position 13:

| (5) | 6 | 7 | 8 | 9 | 10 (11) | 12 | 13 | (14) |
|---|---|---|---|---|---|---|---|---|
| *iso*-Butyryl- | His— | Pro— | Phe— | His— | Sta— | Leu— | Leu— | $OCH_3$ |
| 〃 | 〃 | 〃 | 〃 | 〃 | 〃 | 〃 | –Tyr—$NH_2$ | |
| *tert*-Butyloxycarbonyl- | X | X | 〃 | 〃 | 〃 | 〃 | –Trp—$NH_2$ | |
| 〃 | 〃 | 〃 | 〃 | 〃 | 〃 | 〃 | –Ala—$OCH_3$ | |
| *iso*-Valeryl- | 〃 | 〃 | 〃 | 〃 | 〃 | 〃 | –Val—Phe–$NH_2$ | |

Variations in position 14:

| (5) | 6 | 7 | 8 | 9 | 10 (11) | 12 | 13 | (14) |
|---|---|---|---|---|---|---|---|---|
| *iso*-Valeryl- | His— | Pro— | Phe— | His— | Sta— | Leu— | Phe— | OH |
| 〃 | 〃 | 〃 | 〃 | 〃 | 〃 | 〃 | 〃 | –$OCH_3$ |
| *iso*-Butyryl- | 〃 | 〃 | 〃 | 〃 | 〃 | 〃 | 〃 | –Gly—OH |
| 〃 | 〃 | 〃 | 〃 | 〃 | 〃 | 〃 | 〃 | –Gly—$OCH_3$ |
| 〃 | 〃 | 〃 | 〃 | 〃 | 〃 | 〃 | Leu—Phe—$NH_2$ | |
| 〃 | 〃 | 〃 | 〃 | 〃 | 〃 | 〃 | Leu—Lys—$NH_2$ | |

*X = amino acid absent from position

The Formula I compounds can be used in the form of salts derived from inorganic or organic acids and bases. Included among such acid addition salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenyl-propionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups can be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others. Water or oil-soluble or dispersible products are thereby obtained.

The novel peptides of the present invention possess an excellent degree of activity in treating renin-associated hypertension and hyperaldosteronism.

For these purposes the compounds of the present invention may be administered parenterally, by inhalation spray, or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, dogs, cats, etc., the compounds of the invention are effective in the treatment of humans.

The pharmaceutical compositions may be in the form of sterile injectable preparation, for example as a sterile injectable aqueous or oleagenous suspension. This suspension may be formulated according to the known art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectibles.

The peptides of this invention may also be administered in the form of suppositories for rectal

administration of the drug. These compositions can be prepared by mixing the drug with a suitable nonirritating excipient which is solid at ordinary temperatures but liquid at the rectal temperature and will therefore melt in the rectum to release the drug. Such materials are cocoa butter and polyethylene glycols.

Dosage levels of the order of 2 to 35 grams per day are useful in the treatment of the above indicated conditions. For example, renin-associated hypertension and hyperaldosteronism are effectively treated by the administration of from 30 milligrams to 0.5 grams of the compound per kilogram of body weight per day.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

Thus, in accordance with the present invention there is further provided a pharmaceutical composition for treating renin-associated hypertension and hyperaldosteronism, comprising a pharmaceutical carrier and a therapeutically effective amount of a peptide of the formula:

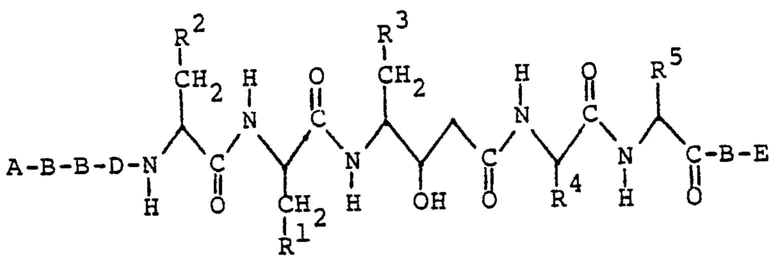

(I.)

wherein A, B, D, $R^1$, $R^2$, $R^3$, $R^4$, $R^5$, and E have the same meaning as recited further above for Formula I; wherein all of the asymmetric carbon atoms have an *S* configuration, except those of sustituents B and D, which may also have the *R* configuration; and a pharmaceutically acceptable salt thereof.

Also, in accordance with the present invention there is still further provided a method of treating renin-associated hypertension and hyperaldosteronism, comprising administering to a patient in need of such treatment, a therapeutically effective amount of a peptide of the formula:

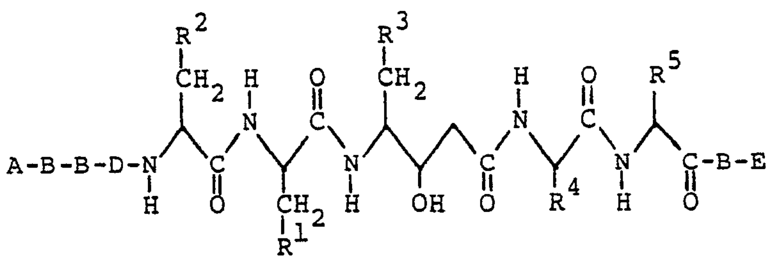

(I.)

wherein A, B, D, $R^1$, $R^2$, $R^3$, $R^4$, $R^5$, and E have the same meaning as recited further above for Formula I; wherein all of the asymmetric carbon atoms have an *S* configuration, except those of sustituents B and D, which may also have the *R* configuration; and a pharmaceutically acceptable salt thereof.

The renin inhibitory novel peptides of the present invention may also be utilized in diagnostic methods for the purpose of establishing the significance of renin as a causative or contributory factor in hypertension or hyperaldosteronism in a particular patient. For this purpose the novel peptides of the present invention may be administered in a single dose of from 0.1 to 10 mg per kg of the body weight.

Both in vivo and in vitro methods may be employed. In the in vivo method, a novel peptide of the present invention is administered to a patient, preferably by intravenous injection, although parenteral administration is also suitable, at a hypotensive dosage level and as a single dose, and there may result a transitory fall in blood pressure. This fall in blood pressure, if it occurs, indicates a supranormal plasma renin levels.

An in vitro method which may be employed involves incubating a body fluid, preferably plasma, with a novel peptide of the present invention and, after deproteinization, measuring the amount of angiotensin II produced in nephrectomized, pentolinium-treated rats. Another in vitro method involves mixing the plasma or other body fluid with a novel peptide of the present invention and injecting the mixture into a test animal. The difference in pressor response with and without added peptide is a measure of the renin content of the plasma.

Pepstatin may be employed in the methods described above as an active control. See, e.g., U.S. Patent Nos. 3,784,686 and 3,873,681 for a description of the use of pepstatin in diagnostic methods of this type.

The novel peptides of the present invention may be prepared in accordance with well-known procedures for preparing peptides from their constituent amino acids, which will be described in more detail below.

A method of preparing a peptide of the formula:

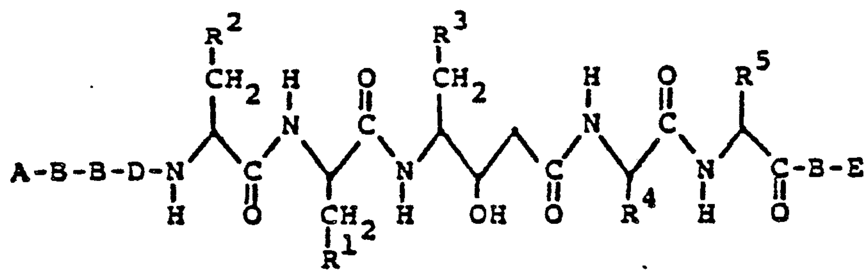

(I)

wherein:

A is hydrogen;

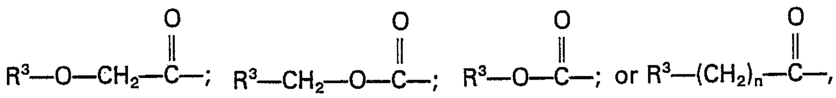

or

where n is 0 to 5 and $R^3$ has the same meaning as set out further below, and may additionally be hydrogen;

B is absent; glycyl; sarcosyl; or

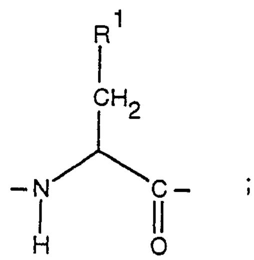

D is absent; or

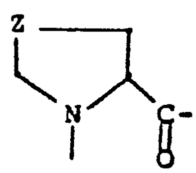

where Z is $(CH_2)_n$ and n is 1 or 2; or —S—;

$R^1$ is hydrogen; $C_{1-4}$ alkyl; hydroxy $C_{1-4}$ alkyl; phenyl; phenyl mono-substituted with a member selected from the group consisting of methyl, trifluoromethyl, hydroxy, methoxy, fluoro, chloro, bromo, and iodo; indolyl; 4-imidazolyl; amine $C_{2-4}$ alkyl; guanidyl $C_{2-3}$ alkyl; or methylthiomethyl;

$R^2$ is hydrogen; $C_{1-4}$ alkyl; phenyl; phenyl mono-substituted with a member selected from the group consisting of methyl, trifluoromethyl, hydroxy, methoxy, fluoro, chloro, bromo, and iodo; or indolyl;

$R^3$ is $C_{3-6}$ alkyl; $C_{3-7}$ cycloalkyl; phenyl; or phenyl mono-substituted with a member selected from the group consisting of methyl, trifluoromethyl, hydroxy, methoxy, fluoro, chloro, bromo, and iodo;

$R^4$ is hydrogen; or

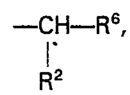

where $R^6$ is hydrogen; $C_{1-4}$ alkyl; hydroxy; or $C_{3-7}$ cycloalkyl; and $R^2$ is as defined above;

$R^5$ is hydrogen;

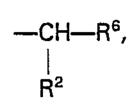

where $R^6$ is hydrogen, $C_{1-4}$ alkyl, hydroxy, or $C_{3-7}$ cycloalkyl, and $R^2$ is as defined above; or $—CH_2—R^7$, where $R^7$ is 4-imidazolyl, amino $C_{2-4}$ alkyl, 2-, 3- or 4-pyridyl or guanidyl $C_{2-3}$ alkyl; and

E is $OR^8$; $NHR^8$, or $N(R^8)_2$, where each $R^8$ may be the same or different and is hydrogen or $C_{1-4}$ alkyl; wherein all of the asymmetric carbon atoms have an *S* configuration, except those of substituents B and D, which may also have the *R* configuration; and a pharmaceutically acceptable salt thereof said peptide being comprised of from five to nine amino acids identified as I through IX, amino acids AA I being at the C-terminus of aid peptide, to which substituent E is attached, and amino acid AA V through IX being at the N-terminus of said peptide, to which substituent A is attached, comprises the steps of:

(a) treating the desired ester or amide of the C-terminus amino acid AA I with the next adjacent amino acid AA II of said peptide, the amino group of said amino acid being protected by a protecting group, in the presence of a condensing agent, whereby a dipeptide of the two amino acids AA I and II is formed;

(b) deprotecting the dipeptide formed in step (a) by removing the protecting group from the amino group of AA II;

(c) treating the dipeptide of AA I and AA II with AA III, the amino group of which is protected by a protecting group, in the presence of a condensing agent, whereby a tripeptide of AA I, AA II and AA III is formed;

(d) deprotecting the tripeptide formed in step (c) by removing the protecting group from the amino group of AA III;

(e) forming a pentapeptide up to a nonapeptide of AA I, through AA V, AA VI, AA VII, AA VIII, or AA IX, by repeating the procedure of step (c) using protected AA IV through protected AA IX;

(f) deprotecting the pentapeptide through nonapeptide formed in step (e) to give the peptide of formula I wherein A is hydrogen; and optionally

(g) treating the pentapeptide through nonapeptide formed in step (f) with $R^3—O—CO—Y$, or $R^3—(CH_2)_n—CO—Y$, where $R^3$ and n are as defined above and Y is an acid halide, anhydride, or other carboxyl activating group, to give the peptide of formula I wherein A is other than hydrogen; and optionally

(h) hydrolyzing the pentapeptide through nonapeptide formed in steps (f) or (g) where an ester of AA I is employed, to give the peptide of formula I wherein E is $OR^8$ and $R^8$ is hydrogen;

said method also comprising, where necessary, protection of sidechain substituents of the component amino acids AA I through AA IX, with deprotection being carried out as a final step;

said method also comprising any combination of the steps set out above, whereby the amino acids I through IX and substituents A and E are assembled in any desired order to prepare the peptide of formula I; and

said method also comprising employment of the steps set out above in a solid phase sequential synthesis, whereby in the initial step the carboxyl group of the selected amino acid is bound to a synthetic resin substrate while the amino group of said amino acid is protected, followed by removal of the protecting group, the succeeding steps being as set out above, the peptide as it is assembled being attached to said synthetic resin substrate; followed by a final step of removing the peptide of formula I from said synthetic resin substrate:

(a) by strong acid cleavage to give E equals $OR^8$ where $R^8$ equals H,

(b) by transesterification with a $C_{1-4}$ alkanol to give E equals $OR^8$ where $R^8$ equals $C_{1-4}$ alkyl;

(c) by ammonolysis with $NH_2R^8$ or $NH(R^8)_2$ to give E equals $NHR^8$ or $N(R^8)_2$ where $R^8$ is hydrogen or $C_{1-4}$ alkyl;

removal of sidechain protecting groups being accomplished either before or after removal of the peptide of formula I from said synthetic resin substrate.

The unusual amino acid, statine, may be prepared in accordance with the procedure described in Rich et. al., *J. Org. Chem.* 43: 3624 (1978).

The novel inhibitory peptides of the present invention are prepared by using the solid phase sequential synthesis technique.

In the following description several abbreviated designations are used for the amino acid components, certain preferred protecting groups, reagents and solvents. The meanings of such abbreviated designations are given below in Table I.

TABLE I

| Abbreviated Designation | Amino Acid |
|---|---|
| Ala | L-alanine |
| Arg | L-arginine |
| Gly | L-glycine |
| His | D or L-histidine |
| Ile | L-isoleucine |
| Leu | L-leucine |
| Lys | L-lysine |
| Met | L-methionine |
| Orn | L-ornithine |
| Phe | L-phenylalanine |
| Pro | D or L-proline |
| Sar | L-sarcosine (N-methylglycine) |
| Ser | L-serine |
| Sta | (3S,4S)-statine |
| Thr | L-threonine |
| Trp | L-tryptophan |
| Tyr | L-tyrosine |
| Val | L-valine |

| Abbreviated Designation | Designated Groups |
|---|---|
| BOC | tert-butyloxycarbonyl |
| CBZ | benzyloxycarbonyl |
| DNP | dinitrophenyl |
| OMe | methyl ester |

| Abbreviated Designation | Activating Groups |
|---|---|
| HBT | 1-hydroxybenzotriazole |

| Abbreviated Designation | Condensing Agents |
|---|---|
| DCCI | dicyclohexylcarbodiimide |
| DPPA | diphenylphosphorylazide |

TABLE I (continued)

| Abbreviated Designation | Reagents |
|---|---|
| TEA | triethylamine |
| TFA | trifluoroacetic acid |

| Abbreviated Designation | Solvents |
|---|---|
| A | ammonium hydroxide (conc.) |
| AcOH | acetic acid |
| C | chloroform |
| DMF | dimethylformamide |
| E | ethyl acetate |
| M | methanol |
| P | pyridine |
| THF | tetrahydrofuran |
| W | water |

The synthesis of the peptides of the present invention by the solid phase technique is conducted in a stepwise manner on chloromethylated resin. The resin is composed of fine beads (20—70 microns in diameter) of a synthetic resin prepared by copolymerization of styrene with 1—2 percent divinylbenzene. The benzene rings in the resin are chloromethylated in a Friedel-Crafts reaction with chloromethyl methyl ether and stannic chloride. The Friedel-Crafts reaction is continued until the resin contains 0.5 to 5 mmoles of chlorine per gram of resin.

The amino acid selected to be the C-terminal amino acid of the linear peptide is converted to its amino protected derivative. The carboxyl group of the selected C-terminal amino acid is bound covalently to the insoluble polymeric resin support, as for example, as the carboxylic ester of the resin-bonded benzyl chloride present in chloromethyl-substituted polystyrene-divinylbenzene resin. After the amino protecting group is removed, the amino protected derivative of the next amino acid in the sequence is added along with a coupling agent, such as dicyclohexylcarbodiimide. The amino acid reactant may be employed in the form of a carboxyl-activated amino acid such as ONp ester an amino acid azide, and the like. Deprotection and addition of successive amino acids is performed until the desired linear peptide is formed.

The selection of protecting groups is, in part, dictated by particular coupling conditions, in part by the amino acid and peptide components involved in the reaction.

Amino-protecting groups ordinarily employed include those which are well known in the art, for example, urethane protecting substituents such as benzyloxy-carbonyl (carbobenzoxy), p-methoxycarbobenzoxy, p-nitrocarbobenzoxy, t-butyoxycarbonyl, and the like. It is preferred to utilize t-butyloxycarbonyl (BOC) for protecting the α-amino group in the amino acids undergoing reaction at the carboxyl end of said amino acid. The BOC protecting group is readily removed following such coupling reaction and prior to the subsequent step by the relatively mild action of acids (i.e. trifluoroacetic acid, or hydrogen chloride in ethyl acetate).

The OH group of Thr and Ser can be protected by the Bzl group and the -amino group of Lys can be protected by the INOC group or the 2-chlorobenzyloxycarbonyl (2-CL-CBZ) group. Neither group is affected by TFA, used for removing BOC protecting groups. After the peptide is formed, the protective groups, such as 2-Cl-CBZ and Bzl, can be removed by treatment with HF or by catalytic hydrogenation.

After the peptide has been formed on the solid phase resin, it may be removed from the resin by a variety of methods which are well known in the art. For example the peptide may be cleaved from the resin with hydrazine, by ammonia in methanol, or by methanol plus a suitable base.

Preparation of the novel inhibitory peptides of the present invention utilizing the solid phase technique is illustrated in the following examples.

Example 1

N-Isobutyryl-L-histidyl-L-prolyl-L-phenylalanyl-L-histidyl-(3S,4S)-statyl-L-valyl-L-histidyl-L-glycyl amide

The title peptide is prepared by standard solid phase methodology, as described in Erickson and Merrifield, *Proteins,* 3rd et., 2:257-527, 1976, using a Beckman Model 990B peptide synthesizer to carry out the operations according to the attached programs. The starting polymer is BOC-Gly esterified to 2% cross-linked polystyrene-divinylbenzene (6 mmol, 5.00 g). The $N^{\alpha}$-BOC derivatives of His-DNP, Val, Sta, His-DNP, Phe, and Pro are coupled using dicyclohexylcarbodiimide with an equivalent of the additive 1-hydroxybenzotriazole hydrate. The Sta is prepared in accordance with Rich et al., *J. Org. Chem.* 43:3624, 1978. The BOC-group is removed with 40% trifluoroacetic acid. A coupling of 60 minutes followed by a recoupling of 120 minutes (2.5 equivalents each time of BOC-amino acid) are used for each amino acid, except for Sta. These coupling times have been previously demonstrated to give complete coupling (as judged by the method of Kaiser) in this sequence. In order to conserve the amounts of Sta employed, an initial coupling using 1.08 equivalents of $N^{\alpha}$-BOC-Sta (in 20 ml of 1:1 $CH_2Cl_2$/DMF) for 72 hrs gives approximately 95% complete reaction. The addition of an additional 0.12 equivalents of $N^{\alpha}$-BOC-Sta plus an equal amount of DCCI to the stirring suspension gives complete coupling after an additional 18 hrs. The N-terminal isobutyryl group is coupled for 60 minutes as the symmetrical anhydride generated in situ from 5.0 equivalents of isobutyric acid and 2.5 equivalents of DCCI. This is followed by a recoupling for 120 minutes using 2.5 equivalents of isobutyric acid, HBT, and DCCI. The DNP protecting groups on His are removed in the final program using two 25-minute treatments with 10% thiophenyl in DMF. The finished resin-peptide is dried and suspended in 30 ml methanol containing 1 g ammonium acetate in a 100 ml pressure bottle containing a magnetic stirring bar. The suspension is cooled under nitrogen to −20°C, and anhydrous ammonia is bubbled through the suspension until it is saturated. The pressure bottle is then closed and allowed to warm to room temperature. The suspension is stirred for 72 hrs, after which the pressure valve is carefully opened, allowing the ammonia to escape. The suspension of beads in orange-red solution is filtered and the beads washed. The filtrate and washes are evaporated and the solid dried. This crude product is then partitioned between water (200 ml) and ethyl acetate (200 ml). The ethyl acetate layer is washed three times with 100 ml of a 1% citric acid solution. The product and the acid layer are neutralized with solid sodium hydrogencarbonate. A yellow oil precipitates from the water when it becomes basic. The now basic water solution and precipitated oil are extracted four times with 100 ml of dichloromethane, and the organic layers are dried and evaporated to give 6.4 g of crude yellow solid. This solid is dissolved in 50 ml of 80:20:2.5, chloroform/methanol/water and loaded onto a silica column (E. Merck No. 9385 silica, 0.040—0.063 mm particle size) 8.9 × 47 cm (ca. 1500 g silica gel) which has been packed in the same solvent. The column is eluted at 18 ml/min with the same solvent and, after 2700 ml, fractions are collected (27 ml each). The pure product is identified by TLC of the fractions. These fractions are combined and evaporated to a light yellow oil. The oil is dissolved in 300 ml of water. The solution is filtered (10 μ) and freeze dried to give the final product.

TLC: 50:40:10 C/M/A Rf = 0.66

For all of the above no impurities are detected at the 2% level, i.e., the product is greater than 99% pure.

HPLC: Greater than 99% single peak.

| Spinco: | | |
|---|---|---|
| | His | 2.02 |
| | Pro | 1.00 |
| | Phe | 0.99 |
| | Val | 0.99 |
| | Gly | 0.99 |

SCHEDULE OF STEPS FOR 6 MMOL RUN

| Step | Solvent/Reagent | Vol. (ml) | Mix time (min) |
|---|---|---|---|
| Coupling Programme 1 | | | |
| 1 | $CH_2Cl_2$ | 6 × 60 | 2 |
| 2 | 40% TFA in $CH_2Cl_2$ | 1 × 60 | 2 |
| 3 | 40% TFA $CH_2Cl_2$ | 1 × 60 | 25 |
| 4 | $CH_2Cl_2$ | 3 × 60 | 2 |
| 5 | 10% TEA in $CH_2Cl_2$ | 2 × 60 | 5 |
| 6 | $CH_2Cl_2$ | 3 × 60 | 2 |
| 7 | BOC-amino acid, HBT in 1:1 DMF/$CH_2Cl_2$ | 40 | 5 |

| | | | |
|---|---|---|---|
| 8 | 1.0M DCCl in $CH_2Cl_2$ | 15 | 60 |
| 9 | DMF | 1 × 60 | 2 |
| 10 | MeOH | 2 × 60 | 2 |
| 11 | $CH_2Cl_2$ | 1 × 60 | 2 |

Re-Couple Program 2

| | | | |
|---|---|---|---|
| 1 | $CH_2Cl_2$ | 1 × 60 | 2 |
| 2 | 10% TEA in $CH_2Cl_2$ | 2 × 60 | 5 |
| 3 | $CH_2Cl_2$ | 3 × 60 | 2 |
| 4 | BOC-amino acid, HBT in 1:1 DMF/$CH_2Cl_3$ | 40 | 5 |
| 5 | 1.0M DCCl in $CH_2Cl_2$ | 15 | 120 |
| 6 | DMF | 1 × 60 | 2 |
| 7 | MeOH | 2 × 60 | 2 |
| 8 | $CH_2Cl_2$ | 5 × 60 | 2 |

Program 3 (DNP removal)

| | | | |
|---|---|---|---|
| 1 | $CH_2Cl_2$ | 1 × 60 | 2 |
| 2 | DMF | 2 × 60 | 2 |
| 3 | 10% phenylthiol in DMF | 1 × 60 | 25 |
| 4 | DMF | 1 × 60 | 2 |
| 5 | 10% TEA in $CH_2Cl_2$ | 1 × 60 | 2 |
| 6 | DMF | 2 × 60 | 2 |
| 7 | 10% phenylthiol in DMF | 1 × 60 | 25 |
| 8 | DMF | 3 × 60 | 2 |
| 9 | MeOH | 2 × 60 | 2 |
| 10 | $CH_2Cl_2$ | 2 × 60 | 2 |
| 11 | MeOH | 2 × 60 | 2 |
| 12 | $CH_2Cl_2$ | 2 × 60 | 2 |
| 13 | MeOH | 2 × 60 | 2 |

Example 2—6

Following the standard solid phase methodology described above in Example 1, additional inhibitory peptides of the present invention are prepared, substituting equivalent amounts of the appropriate BOC-amino acid for those utilized in Example 1, and, where necessary, providing N-terminal groups as substitutes for the isobutyryl group in accordance with well established procedures in the art. The peptides prepared are set out in the following table wherein the subscribed numerical value for each amino acid indicates the results of the Spinco amino acid analysis.

| Example No. | Peptide (5) | 6 | 7 | 8 | 9 | 10(11) | 12 | 13 | (14) |
|---|---|---|---|---|---|---|---|---|---|
| 2 | IBU* | His | Pro | Phe | His | Sta | Ile | His | $NH_2$ |
| | | 0.99 | 1.00 | 0.99 | 0.99 | | 1.04 | 0.99 | |
| 3 | | | BOC | Phe | His | Sta | Ile | His | $NH_2$ |
| 4 | | | CBZ | Phe | His | Sta | Ile | His | $NH_2$ |
| 5 | IVA** | His | Pro | Phe | His | Sta | Ile | His | $NH_2$ |
| 6 | IVA | His | Pro | Phe | His | Sta | Leu | His | $NH_2$ |
| | | 0.99 | 1.00 | 1.01 | 0.99 | | 1.01 | 0.99 | |

*IBU = *iso*-butyryl
**IVA = *iso*-valeryl

Example 7

N-Isovaleryl-L-histidyl-L-prolyl-L-phenylalanyl-L-histidyl-(3S,4S)-statyl-L-leucyl-L-phenylalanine amide

The title peptide was prepared by standard solid phase methodology, as described in Erickson and Merrifield, *Proteins,* 3rd et., 2:257—527, 1976, using a Beckman Model 990B peptide synthesizer to carry out the operations according to the attached programs. The starting polymer was BOC-Phe esterified to 2% cross-linked polystryene-divinylbenzene (6 mmol, 5.00 g). The $N^{\alpha}$BOC derivatives of Leu, Sta, His-DNP, Phe, and Pro were coupled using dicyclohexylcarbodiimide with an equivalent of the additive 1-hydroxybenzotriazole hydrate. The Sta was prepared in accordance with Rich et al., *J. Org. Chem.* 43:3624, 1978. The BOC-group was removed with 40% trifluoroacetic acid. A coupling of 60 minutes followed by a recoupling of 120 minutes (2.5 equivalents each time of BOC-amino acid) were used for each amino acid, except for Sta. These coupling times had been previously demonstrated to give complete coupling (as judged by the method of Kaiser) in this sequence. In order to conserve the amounts of Sta employed, an initial coupling using 1.08 equivalents of $N^{\alpha}$-BOC-Sta (in 20 ml of 1:1 $CH_2Cl_2$/DMF) for 72 hrs gave approximately 95% complete reaction. The addition of an additional 0.12 equivalents of $N^{\alpha}$-BOC-Sta plus an equal amount of DCCI to the stirring suspension gave complete coupling after an additional 18 hrs. The N-terminal isovaleryl group was coupled for 60 minutes as the symmetrical anhydride generated in situ from 5.0 equivalents of isovaleric acid and 2.5 equivalents of DCCI. This was followed by a recoupling for 120 minutes using 2.5 equivalents of isovaleric acid, HBT, and DCCI. The DNP protecting groups on His were removed in the final program using two 25-minute treatments with 10% thiophenol in DMF. The finished resin-peptide was dried and suspended in 30 ml methanol containing 1 g ammonium acetate in a 100 ml pressure bottle containing a magnetic stirring bar. The suspension was cooled under nitrogen to −20°C, and anhydrous ammonia was bubbled through the suspension until it was saturated. The pressure bottle was then closed and allowed to warm to room temperature. The suspension was stirred for 72 hrs, after which the pressure valve was carefully opened, allowing the ammonia to escape. The suspension of beads in orange-red solution was filtered and the beads washed. The filtrate and washes were evaporated and the solid dried. This crude product was then partitioned between water (200 ml) and ethyl acetate (200 ml). The ethyl acetate layer was washed three times with 100 ml of a 1% citric acid solution. The product and the acid layer were neutralized with solid sodium hydrogencarbonate. A yellow oil precipitated from the water when it became basic. The now basic water solution and precipitated oil were extracted four times with 100 ml of dichloromethane, and the organic layers were dried and evaporated to give 6.4 g of crude yellow solid. This solid was dissolved in 50 ml of 80:20:2.5:1, chloroform/methanol/water/acetic acid and loaded onto a silica column (E. Merck No. 9385 silica, 0.040—0.063 mm particle size) 8.9 × 47 cm (ca. 1500 g silica gel) which had been packed in the same solvent. The column was eluted at 18 ml/min with the same solvent and, after 2700 ml, fractions were collected (27 ml each). The pure product was found in fractions 70—110. These fractions were combined and evaporated to a light yellow oil. The oil was dissolved in 20 ml of acetic acid and 300 ml of water were added. The solution was filtered (10 μ) and freeze dried to give 4.45 g of light yellow powder.

| | | | |
|---|---|---|---|
| TLC: | 80:20:2 | C/M/W | Rf = 0.25 |
| | 80:20:2:1 | C/M/W/AcOH | Rf = 0.25 |
| | 80:20:2 | C/M/A | Rf = 0.51 |
| | 10:5:1:3 | E/P/AcOH/W | Rf = 0.38 |

For all of the above no impurities were detected at the 1% level, i.e., the product was greater than 99% pure.
HPLC: Greater than 99% single peak.

| | | | |
|---|---|---|---|
| Spinco: | His | 1.96 | 89.4% peptide based on mol. wt. 1037.3<br>100% based on for. wt. 1157.4 |
| | Pro | 1.01 | |
| | Phe | 2.00 | |
| | Sta | 1.02 | |
| | Leu | 1.02 | |
| | $NH_3$ | 1.25 | |

$^1H$ NMR: 300 MHz spectrum consistent with structure proposed; no unexpected peaks.

SCHEDULE OF STEPS FOR 6 MMOL RUN

| Step | Solvent/Reagent | Vol. (ml) | Mix time (min) |
|---|---|---|---|
| | Coupling Programme 1 | | |
| 1 | $CH_2Cl_2$ | 6 × 60 | 2 |
| 2 | 40% TFa in $CH_2Cl_2$ | 1 × 60 | 2 |
| 3 | 40% TFA $CH_2Cl_2$ | 1 × 60 | 25 |
| 4 | $CH_2Cl_2$ | 3 × 60 | 2 |
| 5 | 10% TEA in $CH_2Cl_2$ | 2 × 60 | 5 |
| 6 | $CH_2Cl_2$ | 3 × 60 | 2 |
| 7 | BOC-amino acid, HBT in 1:1 DMF/$CH_2Cl_2$ | 40 | 5 |
| 8 | 1.0M DCCI in $CH_2Cl_2$ | 15 | 60 |
| 9 | DMF | 1 × 60 | 2 |
| 10 | MeOH | 2 × 60 | 2 |
| 11 | $CH_2Cl_2$ | 1 × 60 | 2 |
| | Re-Couple Program 2 | | |
| 1 | $CH_2Cl_2$ | 1 × 60 | 2 |
| 2 | 10% TEA in $CH_2Cl_2$ | 2 × 60 | 5 |
| 3 | $CH_2Cl_2$ | 3 × 60 | 2 |
| 4 | BOC-amino acid, HBT in 1:1 DMF/$CH_2Cl_2$ | 40 | 5 |
| 5 | 1.0M DCCI in $CH_2Cl_2$ | 15 | 120 |
| 6 | DMF | 1 × 60 | 2 |
| 7 | MeOH | 2 × 60 | 2 |
| 8 | $CH_2Cl_2$ | 5 × 60 | 2 |

Program 3 (DNP removal)

| | | | |
|---|---|---|---|
| 1 | $CH_2Cl_2$ | 1 × 60 | 2 |
| 2 | DMF | 2 × 60 | 2 |
| 3 | 10% phenylthiol in DMF | 1 × 60 | 25 |
| 4 | DMF | 1 × 60 | 2 |
| 5 | 10% TEA in $CH_2Cl_2$ | 1 × 60 | 2 |
| 6 | DMF | 2 × 60 | 2 |
| 7 | 10% phenylthiol in DMF | 1 × 60 | 25 |
| 8 | DMF | 3 × 60 | 2 |
| 9 | MeOH | 2 × 60 | 2 |
| 10 | $CH_2Cl_2$ | 2 × 60 | 2 |
| 11 | MeOH | 2 × 60 | 2 |
| 12 | $CH_2Cl_2$ | 2 × 60 | 2 |
| 13 | MeOH | 2 × 60 | 2 |

Example 8—43

Following the standard solid phase methodology described above in Example 7, additional inhibitory peptides of the present invention were prepared, substituting equivalent amounts of the appropriate BOC-amino acid for those utilized in Example 7, and, where necessary, providing N-terminal groups as substitutes for the isovaleryl group in accordance with well established procedures in the art. The peptides prepared are set out in the following table wherein the subscribed numerical value for each amino acid indicates the results of the Spinco amino acid analysis.

| Example No. | | Peptide (5) | 6 | 7 | 8 | 9 | 10(11) | 12 | 13 | (14) |
|---|---|---|---|---|---|---|---|---|---|---|
| 8 | | H - | His - 1.01 | Pro - 1.04 | Phe - 1.01 | His - 1.01 | Sta - 0.97 | Leu - 0.95 | Phe - 1.01 | $NH_2$ |
| 9 | | BOC - | His - 1.02 | Pro - 1.04 | Phe - 0.98 | His - 1.02 | Sta - | Leu - 0.98 | Phe - 0.98 | $NH_2$ |
| 10 | | IBU*- | His - | Pro - | Phe - | His - | Sta - | Leu - | Phe - | $NH_2$ |
| 11 | BOC - | Pro - 1.04 | His - 0.97 | Pro - 1.04 | Phe - 1.02 | His - 0.97 | Sta - 0.99 | Leu - 1.00 | Phe - 1.02 | $NH_2$ |
| 12 | | | H - | Pro - 1.04 | Phe - 1.08 | His - 0.94 | Sta - 0.90 | Leu - 0.95 | Phe - 1.08 | $NH_2$ |
| 13 | | | Acetyl - | Pro - 0.98 | Phe - 1.00 | His - 1.00 | Sta - | Leu - 1.02 | Phe - 1.00 | $NH_2$ |
| 14 | | | BOC - | Pro - 1.09 | Phe - 1.04 | His - 0.95 | Sta - 0.94 | Leu - 0.93 | Phe - 1.04 | $NH_2$ |
| 15 | | Phenoxyacetyl - | | Pro - 1.02 | Phe - 1.00 | His - 0.99 | Sta - | Leu - 0.99 | Phe - 1.00 | $NH_2$ |
| 16 | | | | H - | Phe - 0.98 | His - 1.00 | Sta - | Leu - 1.03 | Phe - 0.99 | $NH_2$ |

| Example No. | Peptide (5) 6 7 8 9 10(11) 12 13 (14) |
|---|---|
| 17 | BOC - Phe - His - Sta - Leu - Phe - $NH_2$<br>0.98 0.99 1.06 0.98 |
| 18 | Cyclopentylcarbonyl - Phe - His - Sta - Leu - Phe - $NH_2$<br>1.02 0.91 1.04 1.02 |
| 19 | IBU - His - Pro - Phe - Ala - Sta - Leu - Phe - $NH_2$<br>0.99 0.98 1.01 0.98 1.02 1.01 |
| 20 | IBU - His - Pro - Ala - Phe - Sta - Leu - Phe - $NH_2$<br>0.95 0.98 0.99 1.04 1.00 1.04 |
| 21 | BOC - His - Pro - Phe - Phe - Sta - Leu - Phe - $NH_2$<br>1.01 0.98 1.00 1.00 1.02 1.00 |
| 22 | IBU - His - Pro - Phe - His - Sta - Gly - Phe - $NH_2$<br>0.94 1.11 1.00 0.94 1.02 1.00 |
| 23 | IBU - His - Pro - Phe - His - Sta - Ala - Phe - $NH_2$<br>1.02 0.96 1.00 1.02 1.00 1.00 |
| 24 | IBU - His - Pro - Phe - His - Sta - Val - Phe - $NH_2$ |
| 25 | BOC - His - Pro - Phe - His - Sta - Ile - Phe - $NH_2$<br>1.01 0.99 1.00 1.01 1.00 0.97 1.00 |
| 26 | BOC - His - Pro - Phe - His - Sta - Phe - Phe - $NH_2$<br>1.04 1.05 0.98 1.04 0.92 0.98 0.98 |
| 27 | IBU - His - Pro - Phe - His - Sta - Cyclohexyl-Ala - Phe - $NH_2$<br>1.00 0.98 1.01 1.00 1.01 |
| 28 | BOC - His - Pro - Phe - His - Sta - Leu - Leu - $NH_2$<br>1.04 1.00 0.97 1.04 1.00 0.98 0.98 |
| 29 | BOC - His - Pro - Phe - His - Sta - Leu - Tyr - $NH_2$<br>0.99 1.04 0.99 0.99 0.97 1.08 1.13 |
| 30 | BOC - Phe - His - Sta - Leu - Tyr - $NH_2$<br>1.00 1.00 1.00 |
| 31 | BOC - Phe - His - Sta - Leu - Ala - $OCH_3$<br>1.00 1.01 1.01 0.98 |
| 32 | IVA**- His - Pro - Phe - His - Sta - Leu - Phe - OH |
| 33 | BOC - His - Pro - Phe - His - Sta - Leu - Phe - $OCH_3$<br>1.01 1.02 0.99 1.01 1.05 0.93 0.99 |
| 34 | IBU - His - Pro - Phe - His - Sta - Leu - Phe - Gly - OH<br>1.02 0.97 1.01 1.02 0.99 1.01 0.98 |
| 35 | IBU - His - Pro - Phe - His - Sta - Leu - Phe - Gly - $OCH_3$<br>1.02 0.97 1.01 1.02 0.99 1.01 0.98 |
| 36 | BOC - His - Pro - Phe - His - Sta - Leu - Leu - Phe - $NH_2$<br>0.98 0.95 1.03 0.98 1.02 1.01 1.01 1.03 |
| 37 | IBU - His - Pro - Phe - His - Sta - Leu - Phe - Lys - $NH_2$<br>1.01 1.03 0.99 1.01 1.00 0.99 1.03 |

| Example No. | Peptide (5) 6 7 8 9 10(11) 12 13 (14) |
|---|---|
| 38 | Sar - Pro - Phe - His - Sta - Leu - Phe - $NH_2$ |
| | 0.99 0.99 1.00 1.01 1.02 0.99 |
| 39 | D - His - Pro - Phe - His - Sta - Leu - Phe - $NH_2$ |
| | 0.98 1.02 1.00 0.98 1.04 1.00 |
| 40 | IVA - His - D - Pro - Phe - His - Sta - Leu - Phe - $NH_2$ |
| | 1.00 0.98 0.99 1.00 1.07 0.99 |
| 41 | BOC - His - Pro - o - I - Phe - His - Sta - Leu - Phe - $NH_2$ |
| | 0.98 1.01 0.98 1.02 1.01 |
| 42 | BOC - His - Pro - Phe - p - I - Phe - Sta - Leu - Phe - $NH_2$ |
| | 0.98 0.97 1.01 1.03 1.01 |
| 43 | IVA - His - Pro - Phe - His - Sta - Leu - Val - Phe - $NH_2$ |
| | 1.01 1.02 1.00 1.01 1.00 0.95 1.00 |

*IBU = *iso*-butyryl
**IVA = *iso*-valeryl

For the peptides prepared above, various analytical methods were carried out to verify the structure of the peptide products. The following table indicates which methods were employed and summarizes the results where practicable.

| Example No. | Analytical Method | | | | |
|---|---|---|---|---|---|
| | TLC[1] (No. of systems) | HPLC[2] | AA[3] | NMR[4] | EA[5] |
| 2 | 97% (3) | 95.7% | X | X | X |
| 6 | 97% (3) | 95.3% | X | X | X |
| 8 | 95% (3) | 93.2% | X | — | — |
| 9 | 95% (2) | 92.4% | X | — | — |
| 10 | 99% (3) | — | X | X | X |
| 11 | 98% (2) | 98.8% | X | — | — |
| 12 | 90% (2) | — | X | — | — |
| 13 | 95% (2) | 94.2% | X | — | X |
| 14 | 98% | 94% | X | — | — |
| 15 | 97% (2) | 90% | X | — | — |
| 16 | 97% (2) | 92.3% | X | — | X |
| 17 | 97% | 88% | X | — | X |
| 18 | 90% | 80% | X | — | X |
| 19 | 98% (2) | 90.5% | X | X | X |
| 20 | 95% (2) | 96.6% | X | X | X |

| Example No. | Analytical Method | | | | |
|---|---|---|---|---|---|
| | TLC[1] | HPLC[2] | AA[3] | NMR[4] | EA[5] |
| | (No. of systems) | | | | |
| 21 | 95% (2) | 95% | X | — | — |
| 22 | 97% | 90% | — | — | X |
| 23 | 99% (2) | 99% | X | X | X |
| 24 | 98% (3) | 96.1% | X | — | X |
| 25 | 98% | — | X | — | — |
| 26 | 95% (2) | 96.7% | X | — | X |
| 27 | 95% (2) | 98% | X | X | X |
| 28 | 95% | 94.1% | X | — | — |
| 29 | 95% (2) | — | X | — | — |
| 30 | 95% | 95% | X | X | — |
| 31 | 95% | 98% | X | X | — |
| 32 | 95% (3) | 80% | — | — | X |
| 33 | 98% | 99% | X | — | — |
| 34 | 98% (2) | 96.7% | X | — | X |
| 35 | 97% (3) | 95.8% | X | — | X |
| 36 | 98% | 98% | X | — | — |
| 37 | 99% (3) | 95.0% | X | — | X |
| 38 | 95% (2) | 97.0% | X | X | X |
| 39 | 95% (2) | 94.7% | X | X | — |
| 40 | 95% (2) | 93.6% | X | X | X |
| 41 | 95% (2) | 95.3% | X | X | X |
| 42 | 95% (2) | 94.0% | X | X | X |
| 43 | 95% (2) | 98.3% | X | X | X |

[1]TLC = thin layer chromatography on silica gel; visualization by reagents which tend to detect peptides; no. of systems refers to number of different solvent mixtures used to elute chromatograms; % refers to estimated purity.
[2]HPLC = high pressure liquid chromatography; detection by ultraviolet absorption at 240 nm or 210 nm; chromatography is reverse phase, i.e., non-polar elution from non-polar support; % refers to purity, but method tends to overestimate non-peptide impurities.
[3]AA = amino acid analysis; peptide are hydrolyzed to their component amino acids, which are then quantitatively measured; values should be 1.00 ± 0.03.
[4]NMR = nuclear magnetic resonance spectroscopy at 300 MHz or 360 MHz for protons; X = spectrum consistent with structure; — = not performed.
[5]EA = elemental analysis for C, H, and N; X = analysis consistent with structure plus added solvent; — = not performed.

Example 44

Hog Renin Inhibition

An assay was carried out in order to determine the inhibitory potency of the peptides of the present invention. The essay measured the inhibitory of hog kidney renin, and was in accordance with the procedure described in Rich et al., *J. Med. Chem.* 23:27, 1980, except that a pH of 7.3 was used. The results of the assay, illustrated in the table below, are expressed as $I_{50}$ values, which refers to the concentration of peptide inhibitor necessary to produce 50% inhibition of renin activity. This $I_{50}$ value is obtained typically by plotting data from four inhibitor concentrations. Pepstatin was used as an active control.

| Peptide | $I_{50}$(M) |
|---|---|
| H-His-Pro-Phe-His-Sta-Leu-Phe-$NH_2$ | $2.0 \times 10^{-8}$ |
| *iso*-Valeryl-His-Pro-Phe-His-Sta-Leu-Phe-$NH_2$ | $3.0 \times 10^{-8}$ |
| Acetyl-Pro-Phe-His-Sta-Leu-Phe-$NH_2$ | $3.7 \times 10^{-8}$ |
| Acetyl-Phe-His-Sta-Leu-Phe-$NH_2$ | |
| BOC-Phe-His-Sta-Leu-Phe-$NH_2$ | $3.6 \times 10^{-8}$ |
| BOC-His-Pro-Phe-His-Sta-Leu-Phe-$NH_2$ | $2.0 \times 10^{-8}$ |
| IBU-His-Pro-Phe-His-Sta-Ala-Phe-$NH_2$ | $3.7 \times 10^{-8}$ |
| IBU-His-Pro-Phe-His-Sta-Ala-Phe-$NH_2$ | $2.0 \times 10^{-8}$ |
| BOC-His-Pro-Phe-His-Sta-Leu-Leu-$OCH_3$ | $2.3 \times 10^{-8}$ |
| BOC-His-Pro-Phe-His-Sta-Leu-Tyr-$NH_2$ | $2.6 \times 10^{-8}$ |
| IBU-His-Pro-Phe-His-Sta-Leu-Phe-Lys-$NH_2$ | $2.3 \times 10^{-8}$ |
| Sar-Pro-Phe-His-Sta-Leu-Phe-$NH_2$ | $6.6 \times 10^{-8}$ |
| D-His-Pro-Phe-His-Sta-Leu-Phe-$NH_2$ | $4.9 \times 10^{-8}$ |
| IVA-His-D-Pro-Phe-His-Sta-Leu-Phe-$NH_2$ | $5.8 \times 10^{-8}$ |
| BOC-His-Pro-*o*-I-Phe-His-Sta-Leu-Phe-$NH_2$ | $4.2 \times 10^{-8}$ |
| BOC-His-Pro-Phe-*p*--I-Phe-Sta-Leu-Phe-$NH_2$ | $8.1 \times 10^{-9}$ |
| IVA-His-Pro-Phe-His-Sta-Leu-Val-Phe-$NH_2$ | $4.6 \times 10^{-8}$ |
| Pepstatin (*iso*-valeryl-Val-Val-Sta-Ala-Sta) | $1.0 \times 10^{-6}$ |

Example 45

Hog Renin and Human Renin Inhibition

In order to illustrate the correlation between hog renin inhibition and human renin inhibition by a single peptide entity, four of the peptide inhibitors evaluated in the hog renin inhibition assay described above in Example 44 were further evaluated in human renin assay based on the method of Haber et al., *J. Clin. Endocrinol.* 29:1349, 1969. This method employs a radioimmunoassay technique to measure the amount of angiotensin I product of renin cleavage of its substrate. Human plasma (lyophilized) was used as the source of human substrate and human renin. $I_{50}$ values were obtained by plotting data at several inhibitor concentrations. The comparative results are illustrated below. Pepstatin was used as an active control.

| | $I_{50}$(M) | |
|---|---|---|
| Peptide | Hog renin | Human renin |
| *iso*-Valeryl-His-Pro-Phe-His-Sta-Leu-Phe-$NH_2$ | $3.0 \times 10^{-8}$ | $1.8 \times 10^{-8}$ |
| *iso*-Butyryl-His-Pro-Phe-His-Sta-Ala-Phe-$NH_2$ | $3.7 \times 10^{-8}$ | $4.2 \times 10^{-8}$ |

| Peptide | $I_{50}$(M) Hog renin | Human renin |
|---|---|---|
| *iso*-Butyryl-His-Pro-Phe-His-Sta-Val-His-Gly-$NH_2$ | $5.4 \times 10^{-7}$ | $7.3 \times 10^{-8}$ |
| *iso*-Valeryl-His-Pro-Phe-His-Sta-Ile-His-$NH_2$ | $9.3 \times 10^{-7}$ | $7.3 \times 10^{-9}$ |
| Pepstatin(*iso*-valeryl-Val-Val-Sta-Ala-Sta) | $1.0 \times 10^{-6}$ | $>>1.0 \times 10^{-6}$ (20% inhibition at $1.0 \times 10^{-6}$) |

**Claims for the Contracting States: BE CH DE FR GB IT LI LU NL SE**

1. A peptide of the formula:

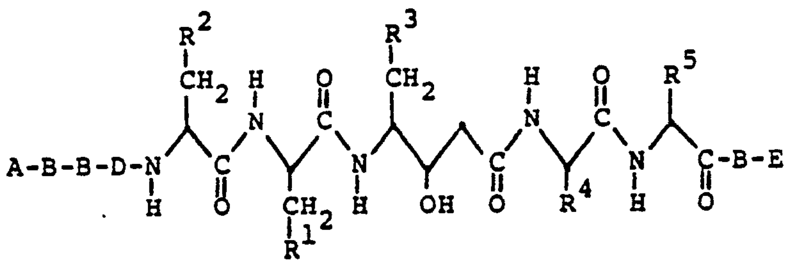

(I.)

wherein:

A is hydrogen;

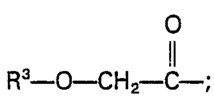 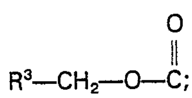 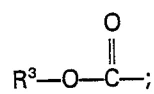 or 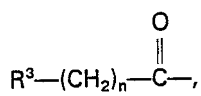

where n is 0 to 5 and $R^3$ has the same meaning as set out further below, and may additionally be hydrogen;

B is absent; glycyl; sarcosyl; or

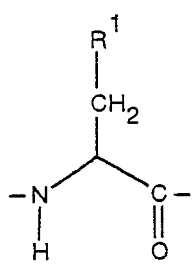 ;

D is absent; or

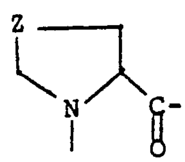

where Z is $(CH_2)_n$ and n is 1 or 2; or —S—;

$R^1$ is hydrogen; $C_{1-4}$ alkyl; hydroxy $C_{1-4}$ alkyl; phenyl; phenyl mono-substituted with a member selected from the group consisting of methyl, trifluoromethyl, hydroxy, methoxy, fluoro, chloro, bromo, and iodo; indolyl; 4-imidazolyl; amine $C_{2-4}$ alkyl; guanidyl $C_{2-3}$ alkyl; or methylthiomethyl;

$R^2$ is hydrogen; $C_{1-4}$ alkyl; phenyl; phenyl mono-substituted with a member selected from the group consisting of methyl, trifluoromethyl, hydroxy, methoxy, fluoro, chloro, bromo, and iodo; or indolyl;

$R^3$ is $C_{3-6}$ alkyl; $C_{3-7}$ cycloalkyl; phenyl; or phenyl mono-substituted with a member selected from the group consisting of methyl, trifluoromethyl, hydroxy, methoxy, fluoro, chloro, bromo, and iodo;

$R^4$ is hydrogen; or

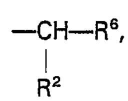

where $R^6$ is hydrogen; $C_{1-4}$ alkyl; hydroxy; or $C_{3-7}$ cycloalkyl; and $R^2$ is as defined above;

$R^5$ is hydrogen;

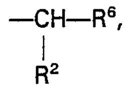

where $R^6$ is hydrogen, $C_{1-4}$ alkyl, hydroxy, or $C_{3-7}$ cycloalkyl, and $R^2$ is as defined above; or $—CH_2—R^7$, where $R^7$ is 4-imidazolyl, amino $C_{2-4}$ alkyl, 2-, 3-, or 4-pyridyl, or guanidyl $C_{2-3}$ alkyl; and

E is $OR^8$; $NHR^8$, or $N(R^8)_2$, where each $R^8$ may be the same or different and is hydrogen or $C_{1-4}$ alkyl; wherein all of the asymmetric carbon atoms have an *S* configuration, except those of substituents B and D, which may also have the *R* configuration; and a pharmaceutically acceptable salt thereof.

2. A peptide according to Claim 1 wherein the peptide is *iso*-butyryl-His-Pro-Phe-His-Sta-Val-His-Gly-$NH_2$.

3. A peptide according to Claim 1 wherein the peptide is *iso*-butyryl-His-Pro-Phe-His-Sta-Ile-His-$NH_2$.

4. A peptide according to Claim 1 wherein the peptide is tert-butyloxycarbonyl-Phe-His-Sta-Ile-His-$NH_2$.

5. A peptide according to Claim 1 wherein the peptide is benzyloxycarbonyl-Phe-His-Sta-Ile-His-$NH_2$.

6. A pharmaceutical composition for treating renin-associated hypertension, comprising a pharmaceutical carrier and a therapeutically effective amount of a peptide of the formula:

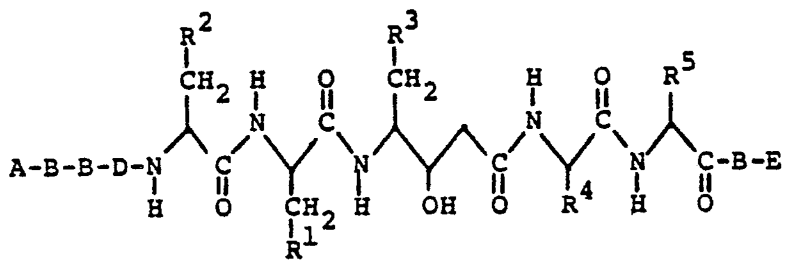

(I.)

wherein:

A is hydrogen;

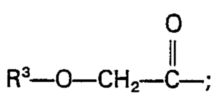; 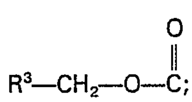; 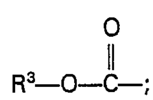; or 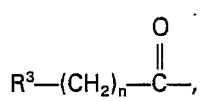,

where n is 0 to 5 and $R^3$ has the same meaning as set out further below, and may additionally be hydrogen;

B is absent; glycyl; sarcosyl; or

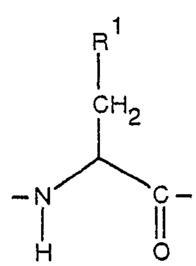;

D is absent; or

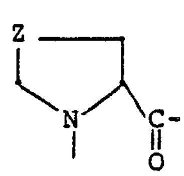

where Z is $(CH_2)_n$ and n is 1 or 2; or —S—;

$R^1$ is hydrogen; $C_{1-4}$ alkyl; hydroxy $C_{1-4}$ alkyl; phenyl; phenyl mono-substituted with a member selected from the group consisting of methyl, trifluoromethyl, hydroxy, methoxy, fluoro, chloro, bromo, and iodo; indolyl; 4-imidazolyl; amine $C_{2-4}$ alkyl; guanidyl $C_{2-3}$ alkyl; or methylthiomethyl;

$R^2$ is hydrogen; $C_{1-4}$ alkyl; phenyl; phenyl mono-substituted with a member selected from the group consisting of methyl, trifluoromethyl, hydroxy, methoxy, fluoro, chloro, bromo, and iodo; or indolyl;

$R^3$ is $C_{3-6}$ alkyl; $C_{3-7}$ cycloalkyl; phenyl; or phenyl mono-substituted with a member selected from the

group consisting of methyl, trifluoromethyl, hydroxy, methoxy, fluoro, chloro, bromo, and iodo;

$R^4$ is hydrogen; or

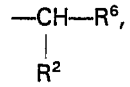

where $R^6$ is hydrogen; $C_{1-4}$ alkyl; hydroxy; or $C_{3-7}$ cycloalkyl; and $R^2$ is as defined above;

$R^5$ is hydrogen;

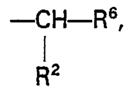

where $R^6$ is hydrogen, $C_{1-4}$ alkyl, hydroxy, or $C_{3-7}$ cycloalkyl, and $R^2$ is as defined above; or $—CH_2—R^7$, where $R^7$ is 4-imidazolyl, amino $C_{2-4}$ alkyl, 2-, 3-, or 4-pyridyl, or guanidyl $C_{2-3}$ alkyl; and

E is $OR^8$; $NHR^8$, or $N(R^8)_2$, where each $R^8$ may be the same or different and is hydrogen or $C_{1-4}$ alkyl; wherein all of the asymmetric carbon atoms have an S configuration, except those of substituents B and D, which may also have the *R* configuration; or a pharmaceutically acceptable salt thereof.

7. A pharmaceutical composition for treating renin-associated hyperaldosteronism, comprising a pharmaceutical carrier and a therapeutically effective amount of a peptide of the formula

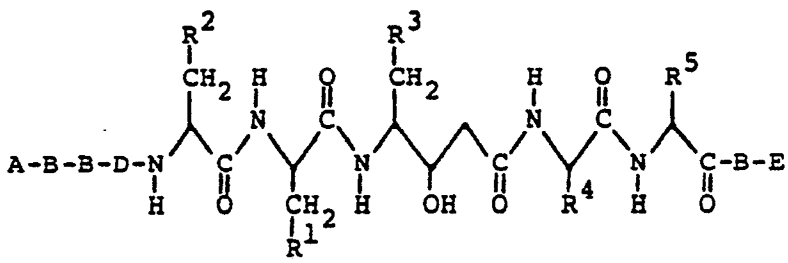 (I.)

wherein:

A is hydrogen;

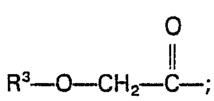; 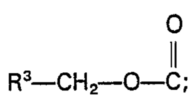; 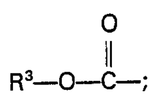; or 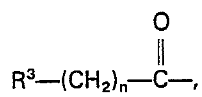,

where n is 0 to 5 and $R^3$ has the same meaning as set out further below, and may additionally be hydrogen;

B is absent; glycyl; sarcosyl; or

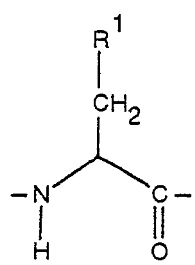 ;

D is absent; or

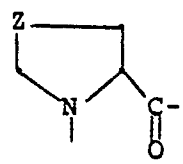

where Z is $(CH_2)_n$ and n is 1 or 2; or —S—;

$R^1$ is hydrogen; $C_{1-4}$ alkyl; hydroxy $C_{1-4}$ alkyl; phenyl; phenyl mono-substituted with a member selected from the group consisting of methyl, trifluoromethyl, hydroxy, methoxy, fluoro, chloro, bromo, and iodo; indolyl; 4-imidazolyl; amine $C_{2-4}$ alkyl; guanidyl $C_{2-3}$ alkyl; or methylthiomethyl;

$R^2$ is hydrogen; $C_{1-4}$ alkyl; phenyl; phenyl mono-substituted with a member selected from the group

consisting of methyl, trifluoromethyl, hydroxy, methoxy, fluoro, chloro, bromo, and iodo; or indolyl;

$R^3$ is $C_{3-6}$ alkyl; $C_{3-7}$ cycloalkyl; phenyl; or phenyl mono-substituted with a member selected from the group consisting of methyl, trifluoromethyl, hydroxy, methoxy, fluoro, chloro, bromo, and iodo;

$R^4$ is hydrogen; or

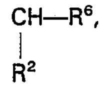

where $R^6$ is hydrogen; $C_{1-4}$ alkyl; hydroxy; or $C_{3-7}$ cycloalkyl; and $R^2$ is as defined above;

$R^5$ is hydrogen;

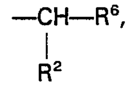

where $R^6$ is hydrogen, $C_{1-4}$ alkyl, hydroxy, or $C_{3-7}$ cycloalkyl, and $R^2$ is as defined above; or $—CH_2—R^7$, where $R^7$ is 4-imidazolyl, amino $C_{2-4}$ alkyl, 2-, 3-, or 4-pyridyl, or guanidyl $C_{2-3}$ alkyl; and

E is $OR^8$; $NHR^8$, or $N(R^8)_2$, where each $R^8$ may be the same or different and is hydrogen or $C_{1-4}$ alkyl; wherein all of the asymmetric carbon atoms have an *S* configuration, except those of substituents B and D, which may also have the *R* configuration; or a pharmaceutically acceptable salt thereof.

**Claims for the Contracting State: AT**

1. A method of preparing a peptide of the formula:

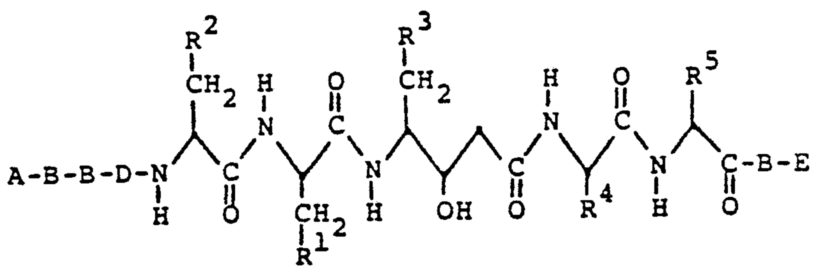

(I )

wherein:

A is hydrogen;

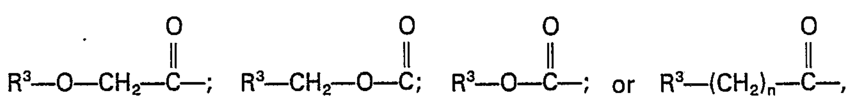

where n is 0 to 5 and $R^3$ has the same meaning as set out further below, and may additionally be hydrogen;

B is absent; glycyl; sarcosyl; or

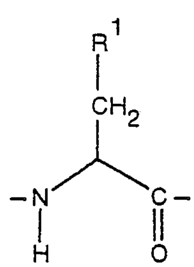

;

D is absent; or

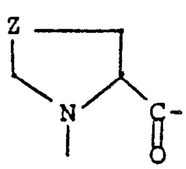

where Z is $(CH_2)_n$ and n is 1 or 2; or —S—;

$R^1$ is hydrogen; $C_{1-4}$ alkyl; hydroxy $C_{1-4}$ alkyl; phenyl; phenyl mono-substituted with a member selected from the group consisting of methyl, trifluoromethyl, hydroxy, methoxy, fluoro, chloro, bromo, and iodo; indolyl; 4-imidazolyl; amine $C_{2-4}$ alkyl; guanidyl $C_{2-3}$ alkyl; or methylthiomethyl;

$R^2$ is hydrogen; $C_{1-4}$ alkyl; phenyl; phenyl mono-substituted with a member selected from the group consisting of methyl, trifluoromethyl, hydroxy, methoxy, fluoro, chloro, bromo, and iodo; or indolyl;

$R^3$ is $C_{3-6}$ alkyl; $C_{3-7}$ cycloalkyl; phenyl; or phenyl mono-substituted with a member selected from the group consisting of methyl, trifluoromethyl, hydroxy, methoxy, fluoro, chloro, bromo, and iodo;

$R^4$ is hydrogen; or

$$-\underset{\displaystyle R^2}{\underset{|}{CH}}-R^6,$$

where $R^6$ is hydrogen; $C_{1-4}$ alkyl; hydroxy; or $C_{3-7}$ cycloalkyl; and $R^2$ is as defined above;

$R^5$ is hydrogen;

$$-\underset{\displaystyle R^2}{\underset{|}{CH}}-R^6,$$

where $R^6$ is hydrogen, $C_{1-4}$ alkyl, hydroxy, or $C_{3-7}$ cycloalkyl, and $R^2$ is as defined above; or $-CH_2-R^7$, where $R^7$ is 4-imidazolyl, amino $C_{2-4}$ alkyl, 2-, 3-, or 4-pyridyl, or guanidyl $C_{2-3}$ alkyl; and

E is $OR^8$; $NHR^8$, or $N(R^8)_2$, where each $R^8$ may be the same or different and is hydrogen or $C_{1-4}$ alkyl; wherein all of the asymmetric carbon atoms have an *S* configuration, except those of substituents B and D, which may also have the *R* configuration; and a pharmaceutically acceptable salt thereof said peptide being comprised of from five to nine amino acids identified as I through IX, amino acid AA I being at the C-terminus of said peptide, to which substituent E is attached, and amino acids AA V through IX being at the N-terminus of said peptide, to which substituent A is attached, comprising the steps of:

(a) treating the desired ester or amide of the C-terminus amino acid AA I with the next adjacent amino acid AA II of said peptide, the amino group of said amino acid being protected by a protecting group, in the presence of a condensing agent, whereby a dipeptide of the two amino acids AA I and II is formed;

(b) deprotecting the dipeptide formed in step (a) by removing the protecting group from the amino group of AA II;

(c) treating the dipeptide of AA I and AA II with AA III, the amino group of which is protected by a protecting group, in the presence of a condensing agent, whereby a tripeptide of AA I, AA II and AA III is formed;

(d) deprotecting the tripeptide formed in step (c) by removing the protecting group from the amino group of AA III;

(e) forming a pentapeptide up to a nonapeptide of AA I, through AA V, AA VI, AA VII, AA VIII, or AA IX, by repeating the procedure of step (c) using protected AA IV through protected AA IX;

(f) deprotecting the pentapeptide through nonapeptide formed in step (e) to give the peptide of formula I wherein A is hydrogen; and optionally

(g) treating the pentapeptide through nonapeptide formed in step (f) with $R^3-O-CO-Y$, or $R^3-(CH_2)_n-CO-Y$, where $R^3$ and n are as defined above and Y is an acid halide, anhydride, or other carboxyl activating group, to give the peptide of formula I wherein A is other than hydrogen; and optionally

(h) hydrolyzing the pentapeptide through nonapeptide formed in steps (f) or (g) where an ester of AA I is employed, to give the peptide of formula I wherein E is $OR^8$ and $R^8$ is hydrogen;

said method also comprising, where necessary, protection of side chain substituents of the component amino acids AA I through AA IX, which deprotection being carried out as a final step;

said method also comprising any combination of the steps set out above, whereby the amino acids I through IX and substituents A and E are assembled in any desired order to prepare the peptide of formula I; and

said method also comprising employment of the steps set out above in a solid phase sequential synthesis, whereby in the initial step the carboxyl group of the selected amino acid is bound to a synthetic resin substrate while the amino group of said amino acid is protected, followed by removal of the protecting group, the succeeding steps being as set out above, the peptide as it is assembled being attached to said synthetic resin substrate; followed by a final step of removing the peptide of formula I from said synthetic resin substrate:

(a) by strong acid cleavage to give E equals $OR^8$ where $R^8$ equals H,

(b) by transesterification with a $C_{1-4}$ alkanol to give E equals $OR^8$ where $R^8$ equals $C_{1-4}$ alkyl; or

(c) by ammonolysis with $NH_2R^8$ or $NH(R^8)_2$ to give E equals $NHR^8$ or $N(R^8)_2$ where $R^8$ is hydrogen or $C_{1-4}$ alkyl;

removal of side chain protecting groups being accomplished either before or after removal of the peptide of formula I from said synthetic resin substrate.

2. The method of claim 1 for preparing the peptide *iso*-butyryl-His-Pro-Phe-His-Sta-Val-His-Gly-$NH_2$.

3. The method of claim 1 for preparing the peptide *iso*-butyryl-His-Pro-Phe-His-Sta-Ile-His-$NH_2$.
4. The method of claim 1 for preparing the peptide tert-butyloxycarbonyl-Phe-His-Sta-Ile-His-$NH_2$.
5. The method of claim 1 for preparing benzyloxycarbonyl-Phe-His-Sta-Ile-His-$NH_2$.

**Patentansprüche für die Vertragsstaaten: BE CH DE FR GB IT LI LU NL SE**

1. Ein Peptid der Formel:

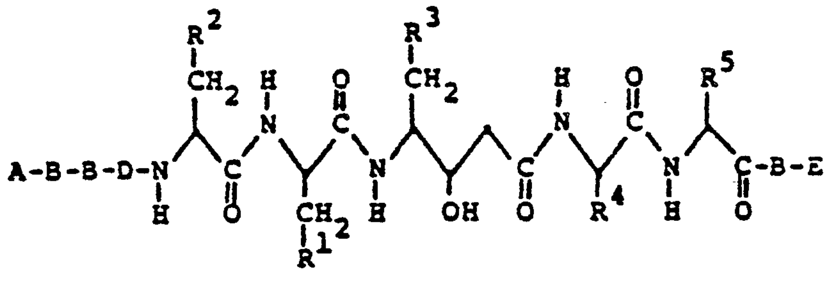

(I.) ,

worin

A Wasserstoff;

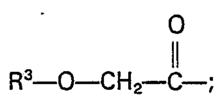 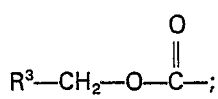 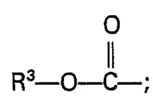 oder 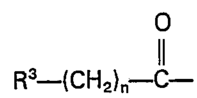 ist,

worin n 0 bis 5 ist und $R^3$ die gleiche Bedeutung wie wieter unten angegeben hat, und zusätzlich Wasserstoff sein kann;

B fehlt; oder Glycyl; Sarcosyl; oder

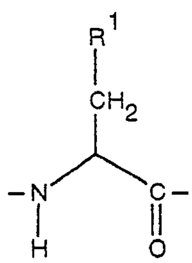

bedeutet;

D fehlt; oder

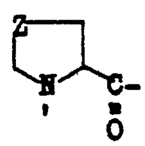

bedeutet, worin Z $(CH_2)_n$ ist, wobei n 1 oder 2 ist; oder —S— bedeutet;

$R^1$ Wasserstoff; $C_{1-4}$-Alkyl; Hydroxy-$C_{1-4}$-alkyl; Phenyl; Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist; Indolyl; 4-Imidazolyl; Amin-$C_{2-4}$-alkyl; Guanidyl-$C_{2-3}$-alkyl; oder Methylthiomethyl; bedeutet;

$R^2$ Wasserstoff; $C_{1-4}$-Alkyl; Phenyl; Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist; oder Indolyl bedeutet;

$R^3$ $C_{3-6}$-Alkyl; $C_{3-7}$-Cycloalkyl; Phenyl; oder Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist, bedeutet;

$R^4$ Wasserstoff; oder

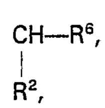

worin $R^6$ Wasserstoff; $C_{1-4}$-Alkyl; Hydroxy; oder $C_{3-7}$-Cycloalkyl ist; und $R^2$ wie oben definiert ist; bedeutet;

$R^5$ Wasserstoff;

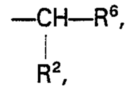

worin $R^6$ Wasserstoff, $C_{1-4}$-Alkyl, Hydroxy oder $C_{3-7}$-Cycloalkyl bedeutet, und $R^2$ wie oben definiert ist; oder $-CH_2-R^7$, worin $R^7$ 4-Imidazolyl, Amino-$C_{2-4}$-alkyl, 2-, 3- oder 4-Pyridyl oder Guanidyl-$C_{2-3}$-alkyl ist, bedeutet; und

E $OR^8$; $NHR^8$ oder $N(R^8)_2$ ist, worin jeder Rest $R^8$ gleich oder verschieden sein kann und Wasserstoff oder $C_{1-4}$-Alkyl bedeutet;

wobei alle asymmetrischen Kohlenstoffatome ein S-Konfiguration, ausgenommen solche in den B- und D-Substituenten, die auch die R-Konfiguration haben können, aufweisen; und ein pharmazeutisch annehmbares Salz davon.

2. Ein Peptid nach Anspruch 1, worin das Peptid Iso-butyryl-His-Pro-Phe-His-Sta-Val-His-Gly-$NH_2$ ist.

3. Ein Peptid nach Anspruch 1, worin das Peptid Iso-butyryl-His-Pro-Phe-His-Sta-Ile-His-$NH_2$ ist.

4. Ein Peptid nach Anspruch 1, worin das Peptid tert.Butyloxycarbonyl-Phe-His-Sta-Ile-His-$NH_2$ ist.

5. Ein Peptid nach Anspruch 1, worin das Peptid Benzyloxycarbonyl-Phe-His-Sta-Ile-His-$NH_2$ ist.

6. Eine pharmazeutische Zusammensetzung für die Behandlung von Renin-assoziierter Hypertension, enthaltend einen pharmazeutischen Träger und eine therapeutisch wirksame Menge eines Peptids der Formel:

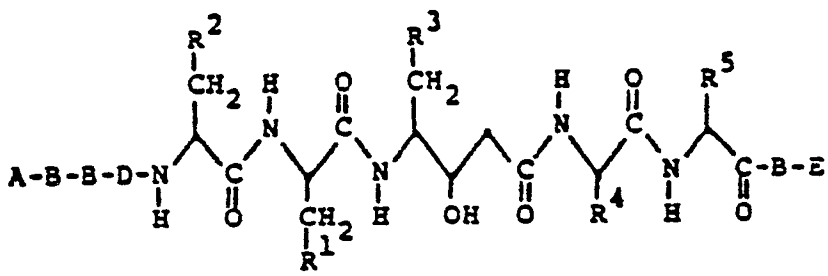

(I.) ,

worin

A Wasserstoff;

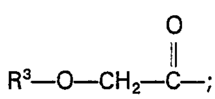 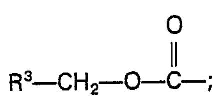 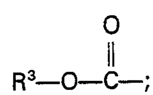 oder 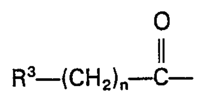 ist,

worin n 0 bis 5 ist und $R^3$ die gleiche Bedeutung wie weiter unten angegeben hat, und zusätzlich Wasserstoff sein kann;

B fehlt; oder Glycyl; Sarcosyl; oder

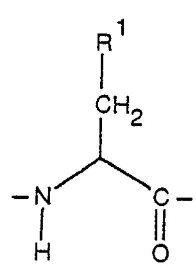

bedeutet;

D fehlt; oder

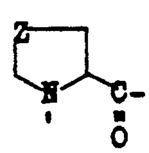

bedeutet, worin Z $(CH_2)_n$ ist, wobei n 1 oder 2 ist; oder —S— bedeutet;

$R^1$ Wasserstoff; $C_{1-4}$-Alkyl; Hydroxy-$C_{1-4}$-alkyl; Phenyl; Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist; Indolyl; 4-Imidazolyl; Amin-$C_{2-4}$-alkyl; Guanidyl-$C_{2-3}$-alkyl; oder Methylthiomethyl; bedeutet;

$R^2$ Wasserstoff; $C_{1-4}$-Alkyl; Phenyl; Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist; oder Indolyl bedeutet;

$R^3$ $C_{3-6}$-Alkyl; $C_{3-7}$-Cycloalkyl; Phenyl; oder Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist, bedeutet;

$R^4$ Wasserstoff; oder

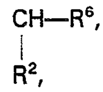

worin $R^6$ Wasserstoff; $C_{1-4}$-Alkyl; Hydroxy; oder $C_{3-7}$-Cycloalkyl ist; und $R^2$ wie oben definiert ist; bedeutet;

$R^5$ Wasserstoff;

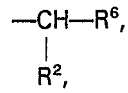

worin $R^6$ Wasserstoff, $C_{1-4}$-Alkyl, Hydroxy oder $C_{3-7}$-Cycloalkyl bedeutet, und $R^2$ wie oben definiert ist; oder $-CH_2-R^7$, worin $R^7$ 4-Imidazolyl, Amino-$C_{2-4}$-alkyl, 2-, 3- oder 4-Pyridyl oder Guanidyl-$C_{2-3}$-alkyl ist, bedeutet; und

E $OR^8$; $NHR^8$ oder $N(R^8)_2$ ist, worin jeder Rest $R^8$ gleich oder verschieden sein kann und Wasserstoff oder $C_{1-4}$-Alkyl bedeutet;

wobei alle asymmetrischen Kohlenstoffatome eine S-Konfiguration, ausgenommen solche in den B- und D-Substituenten, die auch die R-Konfiguration haben können, aufweisen; oder eines pharmazeutisch annehmbaren Salzes davon.

7. Eine pharmazeutische Zusammensetzung für die Behandlung von Renin-assoziiertem Hyperaldosteronismus, enthaltend einen pharmazeutischen Träger und eine therapeutisch wirksame Menge eines Peptids der Formel

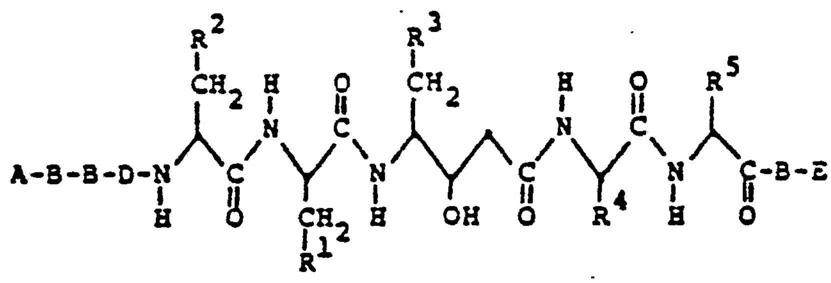

(I.) ,

worin

A Wasserstoff;

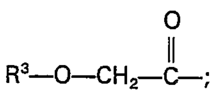 ; 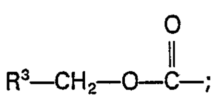 ; 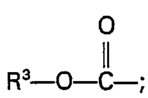 ; oder 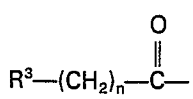 ist,

worin n 0 bis 5 ist und $R^3$ die gleiche Bedeutung wie weiter unten angegeben hat, und zusätzlich Wasserstoff sein kann;

B fehlt; oder Glycyl; Sarcosyl; oder

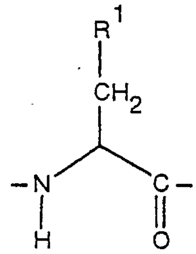

bedeutet;

D fehlt; oder

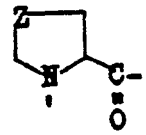

bedeutet, worin Z $(CH_2)_n$ ist, wobei n 1 oder 2 ist; oder —S— bedeutet;

$R^1$ Wasserstoff; $C_{1-4}$-Alkyl; Hydroxy-$C_{1-4}$-alkyl; Phenyl; Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist; Indolyl; 4-Imidazolyl; Amin-$C_{2-4}$-alkyl; Guanidyl-$C_{2-3}$-alkyl; oder Methylthiomethyl; bedeutet;

$R^2$ Wasserstoff; $C_{1-4}$-Alkyl; Phenyl; Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist; oder Indolyl bedeutet;

$R^3$ $C_{3-6}$-Alkyl; $C_{3-7}$-Cycloalkyl; Phenyl; oder Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist, bedeutet;

$R^4$ Wasserstoff; oder

$$\begin{array}{l}CH—R^6,\\ \;|\\ R^2,\end{array}$$

worin $R^6$ Wasserstoff; $C_{1-4}$-Alkyl; Hydroxy; oder $C_{3-7}$-Cycloalkyl ist; und $R^2$ wie oben definiert ist; bedeutet;

$R^5$ Wasserstoff;

$$\begin{array}{l}—CH—R^6,\\ \quad|\\ \quad R^2,\end{array}$$

worin $R^6$ Wasserstoff, $C_{1-4}$-Alkyl, Hydroxy oder $C_{3-7}$-Cycloalkyl bedeutet, und $R^2$ wie oben definiert ist; oder $—CH_2—R^7$, worin $R^7$ 4-Imidazolyl, Amino-$C_{2-4}$-alkyl, 2-, 3- oder 4-Pyridyl oder Guanidyl-$C_{2-3}$-alkyl ist, bedeutet; und

E $OR^8$; $NHR^8$ oder $N(R^8)_2$ ist, worin jeder Rest $R^8$ gleich oder verschieden sein kann und Wasserstoff oder $C_{1-4}$-Alkyl bedeutet;

wobei alle asymmetrischen Kohlenstoffatome ein S-Konfiguration, ausgenommen solche in den B- und D-Substituenten, die auch die R-Konfiguration haben können, aufweisen; oder eines pharmazeutisch annehmbaren Salzes davon.

**Patentansprüche für den Vertragsstaat: AT**

1. Ein Verfahren zur Herstellung eines Peptids der Formel:

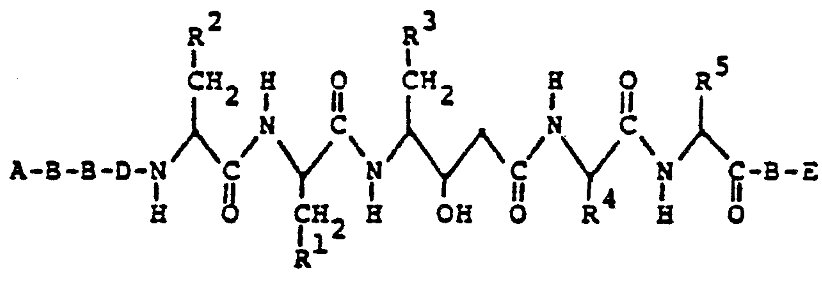

(I.) ,

worin

A Wasserstoff;

$$R^3—O—CH_2—\overset{O}{\overset{\|}{C}}—;\quad R^3—CH_2—O—\overset{O}{\overset{\|}{C}}—;\quad R^3—O—\overset{O}{\overset{\|}{C}}—;\ \text{oder}\ R^3—(CH_2)_n—\overset{O}{\overset{\|}{C}}—\ \text{ist,}$$

worin n 0 bis 5 ist und $R^3$ die gleiche Bedeutung wie weiter unten angegeben hat, und zusätzlich Wasserstoff sein kann;

B fehlt; oder Glycyl; Sarcosyl; oder

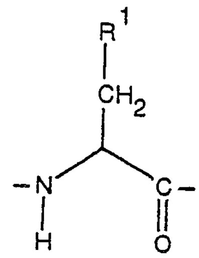

bedeutet;

D fehlt; oder

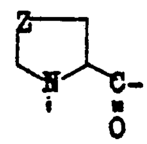

bedeutet, worin Z $(CH_2)_n$ ist, wobei n 1 oder 2 ist; oder —S— bedeutet;

$R^1$ Wasserstoff; $C_{1-4}$-Alkyl; Hydroxy-$C_{1-4}$-alkyl; Phenyl; Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist; Indolyl; 4-Imidazolyl; Amin-$C_{2-4}$-alkyl; Guanidyl-$C_{2-3}$-alkyl; oder Methylthiomethyl; bedeutet;

$R^2$ Wasserstoff; $C_{1-4}$-Alkyl; Phenyl; Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist; oder Indolyl bedeutet;

$R^3$ $C_{3-6}$-Alkyl; $C_{3-7}$-Cycloalkyl; Phenyl; oder Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist, bedeutet;

$R^4$ Wasserstoff; oder

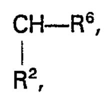

worin $R^6$ Wasserstoff; $C_{1-4}$-Alkyl; Hydroxy; oder $C_{3-7}$-Cycloalkyl ist; und $R^2$ wie oben definiert ist; bedeutet;

$R^5$ Wasserstoff;

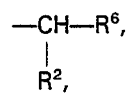

worin $R^6$ Wasserstoff, $C_{1-4}$-Alkyl, Hydroxy oder $C_{3-7}$-Cycloalkyl bedeutet, und $R^2$ wie oben definiert ist; oder $—CH_2—R^7$, worin $R^7$ 4-Imidazolyl, Amino-$C_{2-4}$-alkyl, 2-, 3- oder 4-Pyridyl oder Guanidyl-$C_{2-3}$-alkyl ist, bedeutet; und

E $OR^8$; $NHR^8$ oder $N(R^8)_2$ ist, worin jeder Rest $R^8$ gleich oder verschieden sein kann und Wasserstoff oder $C_{1-4}$-Alkyl bedeutet;

wobei alle asymmetrischen Kohlenstoffatome eine S-Konfiguration, ausgenommen solche in den B- und D-Substituenten, die auch die R-Konfiguration haben können, aufweisen; und eines pharmazeutisch annehmbaren Salzes davon, wobei das genannte Peptid fünf bis neun Aminosäuren enthält, die mit I bis IX bezeichnet sind, Aminosäure AA I sich am C-Terminus des genannten Peptids befindet, an welchen der Substituent E gebunden ist, und Aminosäuren AA V bis AA IX sich am N-Terminus des genannten Peptids befinden, an den der Substituent A gebunden ist, umfassend die Stufen:

(a) der Behandlung des gewünschten Esters oder Amids der C-Terminusaminosäure AA I mit der nächsten benachbarten Aminosäure AA II des genannten Peptids, wobei die Aminogruppe der genannten Aminosäure durch eine Schutzgruppe geschützt ist, in Gegenwart eines Kondensationsmittels, wobei ein Dipeptid der zwei Aminosäuren AA I und AA II gebildet wird;

(b) der Schutzgruppenabspaltung an dem in Stufe (a) gebildeten Dipeptid, durch Entfernung der Schutzgruppe aus der Aminogruppe von AA II;

(c) der Behandlung des Dipeptids von AA I und AA II mit AA III, deren Aminogruppe durch eine Schutzgruppe geschützt ist, in der Gegenwart eines Kondensationsmittels, wobei ein Tripeptid von AA I, AA II und AA III gebildet wird;

(d) der Schutzgruppenabspaltung an dem in Stufe (c) gebildeten Tripeptid, durch Entfernung der Schutzgruppe aus der Aminogruppe von AA III;

(e) der Bildung eines Pentapeptids bis Nonapeptids von AA I bis AA V, AA VI, AA VII, AA VIII oder AA IX, durch Wiederholung der Verhahrensweise von Stufe (c) unter Verwendung von geschützter AA IV bis zu geschützter AA IX;

(f) der Schutzgruppenabspaltung an dem in Stufe (e) gebildeten Pentapeptid bis Nonapeptid, unter Bildung des Peptids der Formel I, worin A Wasserstoff ist; und gegebenenfalls

(g) der Behandlung des in Stufe (f) gebildeten Pentapeptids bis Nonapeptids mit $R^3$—O—CO—Y oder $R^3$—$(CH_2)_n$—CO—Y, worin $R^3$ und n wie oben definiert sind, und Y ein Säurehalogenid, Säureanhydrid oder eine andere Carboxyl aktivierende Gruppe ist, unter Bildung des Peptids der Formel I, worin A eine von Wasserstoff verschiedene Bedeutung hat; und gegebenenfalls

(h) des Hydrolysierens des in Stufen (f) oder (g) unter Anwendung eines Esters von AA I gebildeten Pentapeptids bis Nonapeptids, unter Bildung des Peptids der Formel I, worin E $OR^8$ ist und $R^8$ Wasserstoff ist;

wobei das genannte Verfahren erforderlichenfalls auch den Schutz von Seitenkettensubstituenten der Aminosäurekomponenten AA I bis AA IX unter Vornahme von Schutzgruppenabspaltung als eine letzte Stufe umfaßt;

wobei das genannte Verfahren auch irgendeine Kombination der oben angegebenen Stufen umfaßt, wobei die Aminosäuren AA I bis AA IX und Substituenten A und E in irgendeiner gewünschten Reihenfolge zur Herstellung des Peptids der Formel I zusammengestellt werden; und wobei

das genannte Verfahren auch die Anwendung der oben angegebenen Stufen in einer Festphasenfolgesynthese umfaßt, wobei in der Anfangsstufe die Carboxylgruppe der ausgewählten Aminosäure an ein synthetisches Harzsubstrat gebunden wird, während die Aminogruppe der genannten Aminosäure geschützt ist, anschließend die Schutzgruppe entfernt wird, die Folgestufen wie oben angegeben ausgeführt werden, und das Peptid bei seiner Zusammenstellung an das genannte synthetische Harzsubstrat gebunden ist; worauf eine Endstufe der Entfernung des Peptids der Formel I aus dem genannten synthetischen Harzsubstrat:

(a) durch eine Spaltung mit starker Säure, unter Bildung von E gleich $OR^8$, worin $R^8$ Wasserstoff bedeutet;

(b) durch Umesterung mit einem $C_{1-4}$-Alkanol unter Bildung von E gleich $OR^8$, wobei $R^8$ $C_{1-4}$-Alkyl ist; oder

(c) durch Ammonolyse mit $NH_2R^8$ oder $NH(R^8)_2$ unter Bildung von E gleich $NHR^8$ oder $N(R^8)_2$, worin $R^8$ Wasserstoff oder $C_{1-4}$-Alkyl ist; anschließt,

und wobei die Entfernung von Seitenkettenschutzgruppen entweder vor oder nach der Entfernung des Peptids der Formel I aus dem genannten synthetischen Harzsubstrat erfolgt.

2. Das Verfahren des Anspruchs 1 zur Herstellung des Peptids Iso-butyryl-His-Pro-Phe-His-Sta-Val-His-Gly-$NH_2$.

3. Das Verfahren des Anspruchs 1 zur Herstellung des Peptids Iso-butyryl-His-Pro-Phe-His-Sta-Ile-His-$NH_2$.

4. Das Verfahren des Anspruchs 1 zur Herstellung des Peptids tert.Butyloxycarbonyl-Phe-His-Sta-Ile-His-$NH_2$.

5. Das Verfahren des Anspruchs 1 zur Herstellung von Benzyloxycarbonyl-Phe-His-Sta-Ile-His-$NH_2$.

**Revendications pour les Etats contractants: BE CH DE FR GB IT LI LU NL SE**

1. Un peptide de formule:

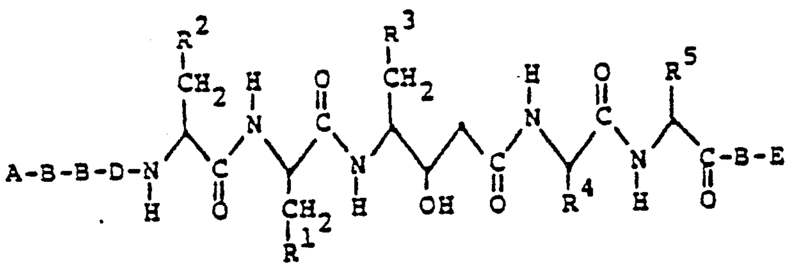

(I.)

dans laquelle:

A est un hydrogène;

$$R^3—O—CH_2—\overset{O}{\overset{\|}{C}}—;\quad R^3—CH_2—O—\overset{O}{\overset{\|}{C}}—;\quad R^3—O—\overset{O}{\overset{\|}{C}}—;\ \text{ou}\ R^3—(CH_2)_n—\overset{O}{\overset{\|}{C}}—,$$

où n est 0 à 5 et $R^3$ a la même signification qu'indiqué ci-après et peut de plus être un hydrogène;

B est absent; un glycyle; un sarcosyle ou

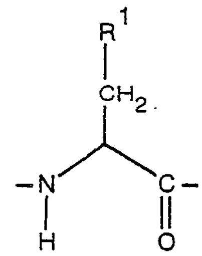

D est absent; ou

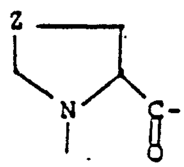

où Z est $(CH_2)_n$ et n est 1 ou 2; ou —S—;

$R^1$ est un hydrogène; un alkyle en $C_{1-4}$; un hydroxyalkyle en $C_{1-4}$; un phényle; un phényle monosubstitué par un composant choisi dans le group constitué par méthyle, trifluorométhyle, hydroxy, méthoxy, fluoro, chloro, bromo et iodo; un indolyle; un 4-imidazolyle; un aminoalkyle en $C_{2-4}$; un guanidyl-alkyle en $C_{2-3}$; ou un méthylthiométhyle;

$R^2$ est un hydrogène; un alkyle en $C_{1-4}$; un phényle; un phényle monosubstitué par un composant choisi dans le groupe constitué par méthyle, trifluorométhyle, hydroxy, méthoxy, fluoro, chloro, bromo et iodo; ou un indolyle;

$R^3$ est un alkyle en $C_{3-6}$; un cycloalkyle en $C_{3-7}$; un phényle; ou un phényle monosubstitué par un composant choisi dans le groupe constitué par méthyle, trifluorométhyle, hydroxy, méthoxy, fluoro, chloro, bromo et iodo;

$R^4$ est un hydrogène; ou

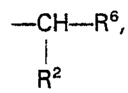

où $R^6$ est un hydrogène; un alkyle en $C_{1-4}$; un hydroxy; ou un cycloalkyle en $C_{3-7}$; et $R^2$ est comme défini ci-dessus;

$R^5$ est un hydrogène;

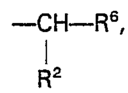

où $R^6$ est un hydrogène, un alkyle en $C_{1-4}$, un hydroxy ou un cycloalkyle en $C_{3-7}$, et $R^2$ est comme défini ci-dessus; ou $—CH_2—R^7$, où $R^7$ est un 4-imidazolyle, un aminoalkyle en $C_{2-4}$, un 2-, 3- ou 4-pyridyle ou un guanidyl-alkyle en $C_{2-3}$; et

E est $OR^8$; $NHR^8$ ou $N(R^8)_2$, où chacun des $R^8$ peut être semblable ou différent et est un hydrogène ou un alkyle en $C_{1-4}$;

où tous les atomes de carbone asymétriques ont une configuration S à l'exception de ceux des substituants B et D qui peuvent également avoir la configuration R; et un sel convenant en pharmacie de celui-ci.

2. Un peptide selon la revendication 1 où le peptide est iso-butyryl-His-Pro-Phe-His-Sta-Val-His-Gly-$NH_2$.

3. Un peptide selon la revendication 1 où le peptide est iso-butyryl-His-Pro-Phe-His-Sta-Ile-His-$NH_2$.

4. Un peptide selon la revendication 1 où le peptide est tert-butoxycarbonyl-Phe-His-Sta-Ile-His-$NH_2$.

5. Un peptide selon la revendication 1 où le peptide est benzyloxycarbonyl-Phe-His-Sta-Ile-His-$NH_2$.

6. Une composition pharmaceutique pour le traitement de l'hypertension associée à la rénine comprenant un support pharmaceutique et une quantité thérapeutique efficace d'un peptide de formule:

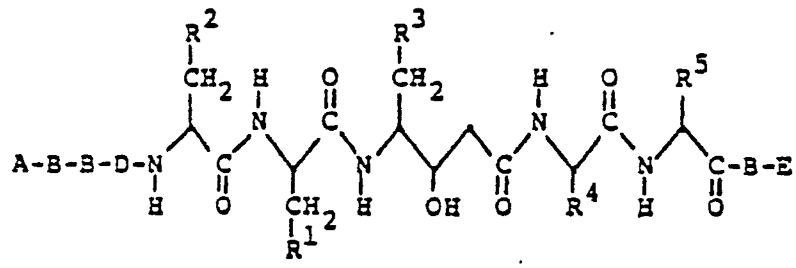

(I.)

dans laquelle:

A est un hydrogène;

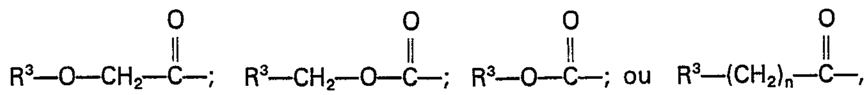

ou

où n est 0 à 5 et $R^3$ a la même signification qu'indiqué ci-après et peut de plus être un hydrogène;

B est absent; un glycyle; un sarcosyle ou

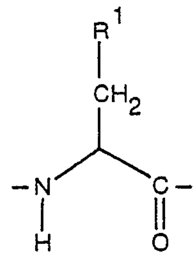

D est absent; ou

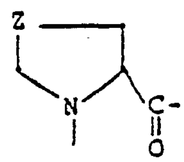

où Z est $(CH_2)_n$ et n est 1 ou 2; ou —S—;

$R^1$ est un hydrogène; un alkyle en $C_{1-4}$; un hydroxyalkyle en $C_{1-4}$; un phényle; un phényle monosubstitué par un composant choisi dans le groupe constitué par méthyle, trifluorométhyle, hydroxy, méthoxy, fluoro, chloro, bromo et iodo; un indolyle; un 4-imidazolyle; un aminoalkyle en $C_{2-4}$; un guanidyl-alkyle en $C_{2-3}$; ou un méthylthiométhyle;

$R^2$ est un hydrogène; un alkyle en $C_{1-4}$; un phényle; un phényle monosubstitué par un composant choisi dans le groupe constitué par méthyle, trifluorométhyle, hydroxy, méthoxy, fluoro, chloro, bromo et iodo; ou un indolyle;

$R^3$ est un alkyle en $C_{3-6}$; un cycloalkyle en $C_{3-7}$; un phényle; ou un phényle monosubstitué par un composant choisi dans le groupe constitué par méthyle, trifluorométhyle, hydroxy, méthoxy, fluoro, chloro, bromo et iodo;

$R^4$ est un hydrogène; ou

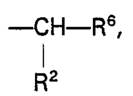

où $R^6$ est un hydrogène; un alkyle en $C_{1-4}$; un hydroxy; ou un cycloalkyle en $C_{3-7}$; et $R^2$ est comme défini ci-dessus;

$R^5$ est un hydrogène;

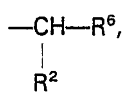

où $R^6$ est un hydrogène, un alkyle en $C_{1-4}$, un hydroxy ou un cycloalkyle en $C_{3-7}$, et $R^2$ est comme défini ci-dessus; ou $—CH_2—R^7$, où $R^7$ est un 4-imidazolyle, un aminoalkyle en $C_{2-4}$, un 2-, 3- ou 4-pyridyle ou un guanidyl-alkyle en $C_{2-3}$; et

E est $OR^8$; $NHR^8$ ou $N(R^8)_2$, où chacun des $R^8$ peut être semblable ou différent et est un hydrogène ou un alkyle en $C_{1-4}$;
où tous les atomes de carbone asymétriques ont une configuration S à l'exception de ceux des substituants B et D qui peuvent également avoir la configuration R; ou un sel convenant en pharmacie de celui-ci.

7. Une composition pharmaceutique pour le traitement de l'hyperaldostéronisme associé à la rénine comprenant un support pharmaceutique et une quantité thérapeutique efficace d'un peptide de formule:

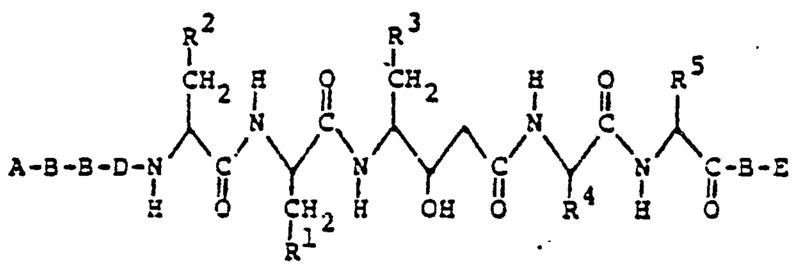

(I.)

dans laquelle:

A est un hydrogène;

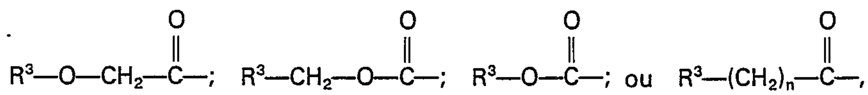

où n est 0 à 5 et $R^3$ a la même signification qu'indiqué ci-après et peut de plus être un hydrogène;

B est absent; un glycyle; un sarcosyle ou

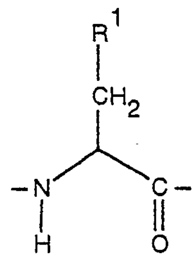

D est absent; ou

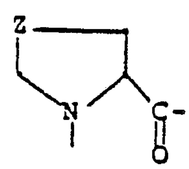

où Z est $(CH_2)_n$ et n est 1 ou 2; ou —S—;

$R^1$ est un hydrogène; un alkyle en $C_{1-4}$; un hydroxyalkyle en $C_{1-4}$; un phényle; un phényle monosubstitué par un composant choisi dans le groupe constitué par méthyle, trifluorométhyle, hydroxy, méthoxy, fluoro, chloro, bromo et iodo; un indolyle; un 4-imidazolyle; un aminoalkyle en $C_{2-4}$; un guanidyl-alkyl en $C_{2-3}$; ou un méthylthiométhyle;

$R^2$ est un hydrogène; un alkyle en $C_{1-4}$; un phényle; un phényle monosubstitué par un composant choisi dans le groupe constitué par méthyle, trifluorométhyle, hydroxy, méthoxy, fluoro, chloro, bromo et iodo; ou un indolyle;

$R^3$ est un alkyle en $C_{3-6}$; un cycloalkyle en $C_{3-7}$; un phényle; ou un phényle monosubstitué par un composant choisi dans le groupe constitué par méthyle, trifluorométhyle, hydroxy, méthoxy, fluoro, chloro, bromo et iodo;

$R^4$ est un hydrogène; ou

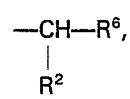

où $R^6$ est un hydrogène; un alkyle en $C_{1-4}$; un hydroxy; ou un cycloalkyle en $C_{3-7}$; et $R^2$ est comme défini ci-dessus;

$R^5$ est un hydrogène;

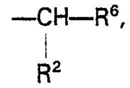

où $R^6$ est un hydrogène, un alkyle en $C_{1-4}$, un hydroxy ou un cycloalkyle en $C_{3-7}$, et $R^2$ est comme défini ci-dessus; ou $—CH_2—R^7$, où $R^7$ est un 4-imidazolyle, un aminoalkyle en $C_{2-4}$, un 2-, 3- ou 4-pyridyle ou un guanidyl-alkyle en $C_{2-3}$; et

E est $OR^8$; $NHR^8$ ou $N(R^8)_2$, où chacun des $R^8$ peut être semblable ou différent et est un hydrogène ou un alkyle en $C_{1-4}$;

où tous les atomes de carbone asymétriques ont une configuration S à l'exception de ceux des substituants B et D qui peuvent également avoir la configuration R; et un sel convenant en pharmacie de celui-ci.

**Revendications pour l'Etat contractant: AT**

1. Un procédé de préparation d'un peptide de formule:

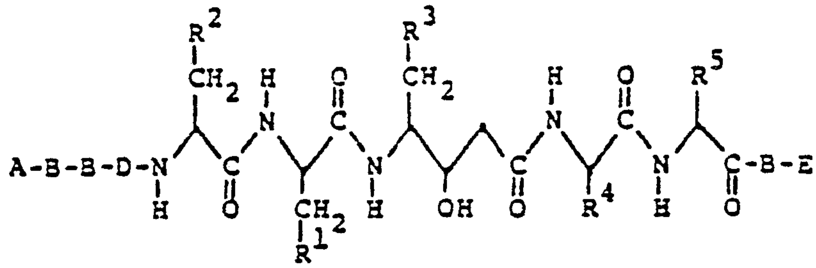

(I)

dans laquelle:

A est un hydrogène;

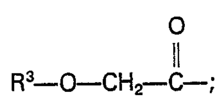 ; 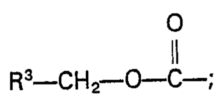 ; 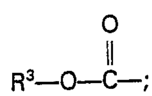 ; ou 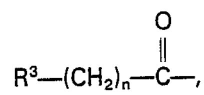 ,

où n est 0 à 5 et $R^3$ a la même signification qu'indiqué ci-après et peut de plus être un hydrogène;

B est absent; un glycyle; un sarcosyle ou

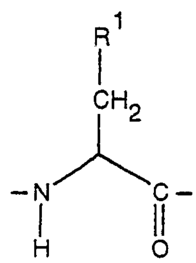

D est absent; ou

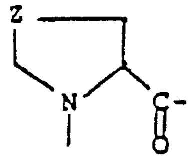

où Z est $(CH_2)_n$ et n est 1 ou 2; ou —S—;

$R^1$ est un hydrogène; un alkyle en $C_{1-4}$; un hydroxyalkyle en $C_{1-4}$; un phényle; un phényle monosubstitué par un composant choisi dans le groupe constitué par méthyle, trifluorométhyle, hydroxy, méthoxy, fluoro, chloro, bromo et iodo; un indolyle; un 4-imidazolyle; un aminoalkyle en $C_{2-4}$; un guanidyl-alkyle en $C_{2-3}$; ou un méthylthiométhyle;

$R^2$ est un hydrogène; un alkyle en $C_{1-4}$; un phényle; un phényle monosubstitué par un composant choisi dans le groupe constitué par méthyle, trifluorométhyle, hydroxy, méthoxy, fluoro, chloro, bromo et iodo; ou un indolyle;

$R^3$ est un alkyle en $C_{3-6}$; un cycloalkyle en $C_{3-7}$; un phényle; ou un phényle monosubstitué par un composant choisi dans le groupe constitué par méthyle, trifluorométhyle, hydroxy, méthoxy, fluoro, chloro, bromo et iodo;

$R^4$ est un hydrogène; ou

$$\begin{array}{c} -CH-R^6, \\ | \\ R^2 \end{array}$$

où $R^6$ est un hydrogène; un alkyle en $C_{1-4}$; un hydroxy; ou un cycloalkyle en $C_{3-7}$; et $R^2$ est comme défini ci-dessus;

$R^5$ est un hydrogène;

$$\begin{array}{c} -CH-R^6, \\ | \\ R^2 \end{array}$$

où $R^6$ est un hydrogène, un alkyle en $C_{1-4}$, un hydroxy ou un cycloalkyle en $C_{3-7}$, et $R^2$ est comme défini ci-dessus; ou $-CH_2-R^7$, où $R^7$ est un 4-imidazolyle, un aminoalkyle en $C_{2-4}$, un 2-, 3- ou 4-pyridyle ou un guanidyl-alkyle en $C_{2-3}$; et

E est $OR^8$; $NHR^8$ ou $N(R^8)_2$, où chacun des $R^8$ peut être semblable ou différent et est un hydrogène ou un alkyle en $C_{1-4}$;

où tous les atomes de carbone asymétriques ont une configuration S à l'exception de ceux des substituants B et D qui peuvent également avoir la configuration R; et un sel convenant en pharmacie de celui-ci. ledit peptide étant constitué de 5 à 9 amino-acides identifiés comme I à IX, l'amino-acide AA I étant à l'extrémité C-terminale dudit peptide, à laquelle le substituant E est fixé, et les amino-acides AA V à IX étant à l'extrémité N-terminale dudit peptide à laquelle le substituant A est attaché, comprend les stades de:

(a) le traitement de l'ester ou de l'amide désiré de l'amino-acide AA I C-terminal avec l'amino-acide juste adjacent AA II dudit peptide, le groupe amino dudit amino-acide étant protégé par un groupe protecteur, en présence d'un agent de condensation pour former un dipeptide des deux amino-acides AA I et II;

(b) la déprotection du dipeptide formé dans le stade (a) par élimination du groupe protecteur du groupe amino de AA II;

(c) le traitement du dipeptide de AA I et AA II avec AA III, dont le groupe amino est protégé par un groupe protecteur, en présence d'un agent de condensation pour former ainsi un tripeptide de AA I, AA II et AA III;

(d) la déprotection du tripeptide formé dans le stade (c) par élimination du groupe protecteur du groupe amino de AA III;

(e) la formation d'un pentapeptide à un nonapeptide de AA I à AA V, AA VI, AA VII, AA VIII ou AA IX par répétition du mode opératoire du stade (c) en utilisant AA IV protégé à AA IX protégé;

(f) la déprotection du pentapeptide à nonapeptide formé dans le stade (e) pour obtenir le peptide de formule I dans laquelle A est un hydrogène; et éventuellement

(g) le traitement du pentapeptide à nonapeptide formé dans le stade (f) avec $R^3-O-CO-Y$ ou $R^3-(CH_2)_n-CO-Y$, où $R^3$ et n sont comme définis ci-dessus et Y est un halogénure d'acide, anhydride d'acide ou un autre groupe d'activation du carboxyle, pour obtenir le peptide de formule I dans laquelle A est autre qu'un hydrogène; et éventuellement

(h) l'hydrolyse du pentapeptide à nonapeptide formé dans le stade (f) ou (g) lorsqu'on emploie un ester de AA I, pour fournir le peptide de formule I dans laquelle E est $OR^8$ et $R^8$ est un hydrogène;

ledit procédé comprenant également, au besoin, la protection des substituants latéraux des amino-acides constitutifs AA I à AA IX, la déprotection étant effectuée comme stade final; ledit procédé comprenant également une combinaison quelconque des stades indiqués ci-dessus, si bien que les amino-acides I à IX et les substituants A et E sont assemblés en un ordre désiré quelconque pour préparer le peptide de formule I; et ledit procédé comprenant également l'emploi des stades mentionnés ci-dessus dans une synthèse séquentielle en phase solide, si bien que dans le stade initial le groupe carboxyle de l'amino-acide choisi est fixé à un substrat de résine synthétique tandis que le groupe amino dudit amino-acide est protégé puis le groupe protecteur est éliminé, les stades suivants étant comme mentionné ci-dessus, le peptide pendant son assemblage étant fixé audit substrat de résine synthétique; suivie d'un stade final d'élimination du peptide de formule I dudit substrat de résine synthétique:

(a) par clivage avec un acide fort pour former E égale $OR^8$ où $R^8$ égale H,

(b) par transestérification avec un alcanol en $C_{1-4}$ pour former E égale $OR^8$ où $R^8$ est égal à un alkyle en $C_{1-4}$;

(c) par ammoniolyse avec $NH_2R^8$ ou $NH(R^8)_2$ pour former E égale $NHR^8$ ou $N(R^8)_2$ où $R^8$ est un hydrogène ou un alkyle en $C_{1-4}$;

l'élimination des groupes protecteurs des chaînes latérales étant effectuée soit avant soit après l'élimination du peptide de formule I dudit substrat de résine synthétique.

2. Le procédé de la revendication 1 pour la préparation du peptide iso-butyryl-His-Pro-Phe-His-Sta-Val-His-Gly-$NH_2$.

3. Le procédé de la revendication 1 pour la préparation du peptide iso-butyryl-His-Pro-Phe-His-Sta-Ile-His-$NH_2$.

4. Le procédé de la revendication 1 pour la préparation du peptide tert-butoxycarbonyl-Phe-His-Sta-Ile-His-$NH_2$.

5. Le procédé de la revendication 1 pour la préparation du peptide de benzyloxycarbonyl-Phe-His-Sta-Ile-His-$NH_2$.